# EUROPEAN PATENT SPECIFICATION

(11) **EP 3 071 230 B1**
(45) Date of publication and mention of the grant of the patent: **08.04.2020**
(21) Application number: 14812084.3
(22) Date of filing: 21.11.2014
(51) Int. Cl.: A61K 39/395, C07K 16/28, A61P 19/02, A61P 37/02

(54) **COMPOSITIONS FOR THE TREATMENT OF RHEUMATOID ARTHRITIS AND METHODS OF USING SAME**
ZUSAMMENSETZUNGEN ZUR BEHANDLUNG VON RHEUMATOIDER ARTHRITIS UND VERFAHREN ZUR VERWENDUNG DAVON
COMPOSITIONS POUR LE TRAITEMENT DE LA POLYARTHRITE RHUMATOÏDE ET MÉTHODES D'UTILISATION DESDITES COMPOSITIONS

(30) Priority: 22.11.2013 US 201361907796 P; 06.06.2014 US 201462008787 P; 05.09.2014 EP 14306366
(43) Date of publication of application: 28.09.2016
(73) Proprietor: Sanofi Biotechnology, 75008 Paris (FR); Regeneron Pharmaceuticals, Inc., Tarrytown, NY 10591 (US)
(72) Inventor: HUANG, Xiaohong, Vernon Hills, IL 60061 (US); JASSON, Martine, 75008 Paris (FR); MARKS, Vanessa, 75008 Paris (FR); RADIN, Allen, Tarrytown, NY 10591 (US); FAN, Chungpeng, Bridgewater, NJ 08807 (US); VAN HOOGSTRATEN, Hubert, NJ 08807 (US); FIORE, Stefano, Bridgewater, NJ 08807 (US); VAN ADELSBERG, Janet, Tarrytown, NY 10591 (US); GENOVESE, Mark, Sunnyvale, CA 94087 (US)
(74) Representative: Lavoix
(86) International application number: PCT/US2014/066856
(87) International publication number: WO 2015/077582

(56) References cited:
- WO-A1-2013/053751
- Anonymous: "NCT01061736 on 2013_11_07: ClinicalTrials.gov Archive", , 7 November 2013 (2013-11-07), XP055178878, Retrieved from the Internet: URL:https://clinicaltrials.gov/archive/NCT 01061736/2013_11_07 [retrieved on 2015-03-24]

### SEQUENCE LISTING

<110> SANOFI Regeneron Pharmaceuticals, Inc.
<120> COMPOSITIONS FOR THE TREATMENT OF RHEUMATOID ARTHRITIS
   AND METHODS OF USING SAME
<130> US2011-131
<140> US 13/648,521
   <141> 2012-10-10
<150> US 61/545,864
   <151> 2012-10-11
<160> 3
<170> PatentIn version 3.3
<210> 1
   <211> 358
   <212> PRT
   <213> Homo sapiens
<220>
   <221> MISC_FEATURE
   <222> (1)..(358)
   <223> extracellular domain og human IL-6R
<400> 1
   Met Val Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
                   245                 250                 255
   Tyr Arg Ala Glu Arg Ser Lys Thr Phe Thr Thr Trp Met Val Lys Asp
               260                 265                 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Glu Asn Glu Val Ser Thr Pro Met Gln Ala Leu Thr Thr
                   325                 330                 335
   Asn Lys Asp Asp Asp Asn Ile Leu Phe Arg Asp Ser Ala Asn Ala Thr
               340                 345                 350
   Ser Leu Pro Val Gln Asp
           355
<210> 2
   <211> 116
   <212> PRT
   <213> Homo sapiens
<220>
   <221> MISC_FEATURE
   <222> (1)..(116)
   <223> heavy chain variable region of sarilumab
<400> 2
   Glu Val Gln Leu Val Glu Ser Gly Gly Gly Leu Val Gln Pro Gly Arg
   1               5                   10                  15
   Ser Leu Arg Leu Ser Cys Ala Ala Ser Arg Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Arg Ile Gly Tyr Ala Asp Ser Val
       50                  55                  60
   Lys Gly Arg Phe Thr Ile Ser Arg Asp Asn Ala Glu Asn Ser Leu Phe
   65                  70                  75                  80
   Leu Gln Met Asn Gly Leu Arg Ala Glu Asp Thr Ala Leu Tyr Tyr Cys
                   85                  90                  95
   Ala Lys Gly Arg Asp Ser Phe Asp Ile Trp Gly Gln Gly Thr Met Val
                   100                 105                 110
   Thr Val Ser Ser
           115
<210> 3
   <211> 107
   <212> PRT
   <213> Homo sapiens
<220>
   <221> MISC_FEATURE
   <222> (1)..(107)
   <223> light chain variable region of sarilumab
<400> 3
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Gly Lys Ala Pro Lys Leu Leu Ile
           35                  40                  45
   Tyr Gly Ala Ser Ser Leu Glu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Ser Tyr Tyr Cys Gln Gln Ala Asn Ser Phe Pro Tyr
                   85                  90                  95
   Thr Phe Gly Gln Gly Thr Lys Leu Glu Ile Lys
               100                 105

## Description

### FIELD OF THE INVENTION

The present invention relates to the field of therapeutic treatment of rheumatoid arthritis. More specifically, the invention relates to the use of interleukin-6 receptor (IL-6R) antagonists, such as anti-IL-6R antibodies combined with disease modifying antirheumatic drugs, to treat rheumatoid arthritis.

### BACKGROUND

It is estimated that approximately 0.5% to 1% of the adult population in North America and Europe is affected by rheumatoid arthritis (RA). RA affects women twice as often as men and the incidence is highest among women over 40 years of age.

The cause(s) of RA are not completely understood. RA is defined by a set of symptoms and is characterized by persistent synovitis and progressive destruction of cartilage and bone in multiple joints. The hallmark of the disease is a symmetric polyarthritis characteristically involving the small joints of the hands and feet. The inflammatory process can also target other organs, characteristically bone marrow (anemia), eye (scleritis, episcleritis), lung (interstitial pneumonitis, pleuritis), heart (pericarditis) and skin (nodules, leukocytoclastic vasculitis). Systemic inflammation is characterized by laboratory abnormalities, such as anemia, elevated erythrocyte sedimentation rate, fibrinogen and C-reactive protein (CRP) and by clinical symptoms of fatigue, weight loss, muscle atrophy in affected joint areas. The presence of polyclonal high-titer rheumatoid factors and anticyclic citrullinated peptide (anti-CCP) antibodies provides evidence of immune dysregulation. It has been estimated that 65% to 70% of RA patients have progressive disease that leads to joint destruction, disability and premature death.

Treatments include both medication and non-pharmacological measures - the goal being to control joint inflammation and prevent joint damage and disability. Non-pharmacological treatment, including physical therapy, splints and braces, occupational therapy, and dietary changes, do not stop the progression of joint destruction. Painkillers and anti-inflammatory drugs, including steroids, suppress symptoms, but do not stop the progression either. Disease-modifying antirheumatic drugs (DMARDs) may slow the progress of the disease.

In addition to the existing treatments improving the symptoms (e.g. inflammation) associated with RA, there is still an important medical need for treatments inhibiting or halting the progression of structural damage (joint damage and destruction) in subjects suffering from RA. There is also a need for treatments further improving the physical function (e.g. dressing and grooming, arising, eating, walking, hygiene, reach, grip and activities) of subjects suffering from RA.

WO2013/053751 discloses the clinical efficacy of sarilumab for treating RA based on ACR scores obtained at 12 weeks.

A clinical trial protocol for the evaluation of sarilumab on top of methotrexate in RA patients has been disclosed under the Clinical trial identifier NTC01061736.

### SUMMARY

The invention relates to an anti-IL6R antibody comprising the heavy chain variable region of SEQ ID NO:2 and the light chain variable region of SEQ ID NO:3 for use in combination with methotrexate for inhibiting the progression of structural damage in a subject suffering from rheumatoid arthritis, wherein an effective amount of the antibody and an effective amount of methotrexate are administered to the subject, and wherein the antibody is administered subcutaneously to the subject between 150 and 200 mg per two weeks.

The invention is further defined by the claims.

The present disclosure provides a method for inhibiting the progression of structural damage in a subject suffering from Rheumatoid Arthritis, comprising administering to the subject an effective amount of sarilumab. Typically, the inhibition of the progression of structural damage is assessed by the change from baseline (BL) in the modified Van der Heijde total Sharp score (mTSS).

The present disclosure also provides a method for improving physical function in a subject suffering from Rheumatoid Arthritis, comprising administering to the subject an effective amount of sarilumab. Typically, improvement of physical function is assessed by the change from baseline (BL) in the Health Assessment Questionnaire Disability Index (HAQ-DI).

The present disclosure provides a method of treating rheumatoid arthritis in a subject in need thereof. The method includes administering to the subject an effective amount of sarilumab (SAR153191) and a member of the group consisting of leflunomide, sulfasalazine and hydroxychloroquine. In certain embodiments, the subject was previously ineffectively treated for rheumatoid arthritis by administering a TNF-α antagonist. Specifically, the subject could have been treated for at least three months with the TNF-α antagonist or could have been intolerant of the TNF-α antagonist. The TNF-α antagonist could be etanercept, infliximab, adalimumab, golimumab or certolizumab. In other embodiments, the subject was previously ineffectively treated for rheumatoid arthritis by administering methotrexate.

In certain aspects, sarilumab and leflunomide are administered to the subject. The leflunomide can be administered orally. The leflunomide can also be administered at between 10 and 20 mg per day to the subject.

In other specific aspects, sarilumab and sulfasalazine are administered to the subject. The sulfasalazine can be administered orally. The sulfasalazine can also be administered at between 1000 to 3000 mg per day to the subject.

In other specific aspects, sarilumab and hydroxychloroquine are administered to the subject. The hydroxychloroquine can be administered orally. The hydroxychloroquine can also be administered at between 200 to 400 mg per day to the subject.

According to any of the embodiments described herein, the subject can be administered sarilumab and/or methotrexate for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39,. 40, 41, 42, 43, 44, 45, 46, 47, 48, 49, 50, 51, 52, 53, 54, 55, 56, 57, 58, 59, 60 or more weeks. In certain embodiments, a subject is administered sarilumab and methotrexate for 12, 16, 24 or 52 weeks or more. In certain embodiments, a subject is administered sarilumab and methotrexate for 52 weeks or more. In particular, sarilumab is administered at 150 or 200 mg every two weeks for at least 52 weeks. Sarilumab is administered subcutaneously at between 150 and 200 mg every two weeks.

In some embodiments, as a result of the treatment, the subject achieves a 20% or 50% improvement in the American College of Rheumatology core set disease index after 12 weeks of treatment. In other embodiments, as a result of the treatment, the subject achieves a 20%, 50% or 70% improvement in the American College of Rheumatology core set disease index after 24 weeks of treatment.

In some embodiments, as a result of the treatment, the subject achieves a lower disease activity score after 12 weeks of treatment than the subject had before treatment. The disease activity score can be less than or equal to 2.6 at 12 weeks. The disease activity score can decrease by greater than 1.2 between the start of treatment and 12 weeks. The disease activity score can be less than or equal to 3.2 at 12 weeks. The disease activity score can decrease by greater than 0.6 between the start of treatment and 12 weeks. The disease activity score can be less than or equal to 5.1 at 12 weeks.

In some embodiments, as a result of the treatment, the subject achieves a lower disease activity score after 24 weeks of treatment than the subject had before treatment. The disease activity score can be less than or equal to 2.6 at 24 weeks. The disease activity score can decrease by greater than 1.2 between the start of treatment and 24 weeks. The disease activity score can be less than or equal to 3.2 at 24 weeks. The disease activity score can decrease by greater than 0.6 between the start of treatment and 24 weeks. The disease activity score can be less than or equal to 5.1 at 24 weeks.

The present disclosure also provides a method of treating rheumatoid arthritis in a subject in need thereof comprising administering to the subject an effective amount of sarilumab and methotrexate, wherein the subject was previously ineffectively treated for rheumatoid arthritis by administering an anti-TNF-a therapeutic. In certain embodiments, the subject was previously ineffectively treated for rheumatoid arthritis by administering methotrexate. The methotrexate can be administered at between 10 to 25 mg per week to the subject.

According to the invention, the subject is a mammal. The mammal can be a human. In certain embodiments, the human is descended from individuals from Asia or the Pacific. Humans descended from individuals from Asia or the Pacific can be administered between 6 and 25 mg per week of methotrexate.

In certain embodiments, the subject was previously ineffectively treated for rheumatoid arthritis by administering a TNF-α antagonist. Specifically, the subject could have been treated for at least three months with the TNF-α antagonist or could have been intolerant of the TNF-α antagonist. The TNF-α antagonist could be etanercept, infliximab, adalimumab, golimumab or certolizumab. In other embodiments, the subject was previously ineffectively treated for rheumatoid arthritis by administering methotrexate.

In some embodiments, as a result of the treatment, the subject achieves a 20% or 50% improvement in the American College of Rheumatology core set disease index after 12 weeks of treatment. In other embodiments, as a result of the treatment, the subject achieves a 20%, 50% or 70% improvement in the American College of Rheumatology core set disease index after 24 weeks of treatment.

In some embodiments, as a result of the treatment, the subject achieves a lower disease activity score after 12 weeks of treatment than the subject had before treatment. The disease activity score can be less than or equal to 2.6 at 12 weeks. The disease activity score can decrease by greater than 1.2 between the start of treatment and 12 weeks. The disease activity score can be less than or equal to 3.2 at 12 weeks. The disease activity score can decrease by greater than 0.6 between the start of treatment and 12 weeks. The disease activity score can be less than or equal to 5.1 at 12 weeks.

In some embodiments, as a result of the treatment, the subject achieves a lower disease activity score after 24 weeks of treatment than the subject had before treatment. The disease activity score can be less than or equal to 2.6 at 24 weeks. The disease activity score can decrease by greater than 1.2 between the start of treatment and 24 weeks. The disease activity score can be less than or equal to 3.2 at 24 weeks. The disease activity score can decrease by greater than 0.6 between the start of treatment and 24 weeks. The disease activity score can be less than or equal to 5.1 at 24 weeks.

The disclosure also provides a pharmaceutical composition comprising an effective amount of sarilumab and a member of the group consisting of leflunomide, sulfasalazine and hydroxychloroquine. Sarilumab could be present at between 50 and 150 mg per dose or between 100 and 200 mg per dose.

In certain specific aspects, the composition includes sarilumab and leflunomide. The leflunomide can be present in an oral dosage form. The leflunomide can be present in the composition at between 10 and 20 mg per dose.

In other specific aspects, the composition includes sarilumab and sulfasalazine. The sulfasalazine can be present in an oral dosage form. The sulfasalazine can be present in the composition at between 1000 to 3000 mg per day to the subject.

In other specific aspects, the composition includes sarilumab and hydroxychloroquine. The hydroxychloroquine can be present in an oral dosage form. The hydroxychloroquine can be present in the composition at between 200 to 400 mg per day to the subject.

In certain aspects, the disclosure also provides a method of treating rheumatoid arthritis in a subject previously treated by administering methotrexate, leflunomide, sulfasalazine and/or hydroxychloroquine, comprising administering to the subject an effective amount of sarilumab.

In one embodiment, the subject was previously ineffectively treated for rheumatoid arthritis by administering methotrexate, leflunomide, sulfasalazine and/or hydroxychloroquine.

In another aspect, methotrexate, leflunomide, sulfasalazine and/or hydroxychloroquine is administered together with sarilumab.

In another embodiment, sarilumab and methotrexate are administered together.

In one embodiment, methotrexate is administered between 6 to 25 mg per week.

In other embodiments, sarilumab is administered at 150 mg per two weeks. In other embodiments, sarilumab is administered at 200 mg per two weeks.

In certain embodiments, the subject achieves a 20% improvement in the American College of Rheumatology core set disease index (ACR20) after 12 weeks of treatment. In other embodiments, the subject achieves a 50% improvement in the American College of Rheumatology core set disease index (ACR50) after 12 weeks of treatment. In other embodiments, the subject achieves a 70% improvement in the American College of Rheumatology core set disease index (ACR70) after 12 weeks of treatment.

In certain embodiments, the subject was previously ineffectively treated for rheumatoid arthritis by administering a TNF-α antagonist. In one embodiment, the subject has been treated with an anti-TNF-α antagonist for at least 3 months in the last 2 years or the subject was intolerant to at least one TNF-α antagonist. In another embodiment, the TNF-α antagonist is a biologic anti-TNF-α. In another embodiment, the TNF-α antagonist is selected from the group consisting in etanercept, infliximab, adalimumab, golimumab and/or certolizumab pegol.

In other aspects, the disclosure provides a pharmaceutical composition comprising an effective amount of sarilumab and a member of the group consisting of methotrexate, leflunomide, sulfasalazine and hydroxychloroquine.

In yet other aspects, the disclosure provides a combination of: a pharmaceutical composition comprising sarilumab, and a pharmaceutical composition comprising methotrexate, leflunomide, sulfasalazine or hydroxychloroquine for sequential or simultaneous use as a medicament.

### DETAILED DESCRIPTION

The disclosure provides pharmaceutical compositions and methods of using these compositions for the treatment of rheumatoid arthritis (RA) and the improvement of at least one symptom of RA. These compositions include at least one antibody that specifically binds human interleukin-6 receptor (hIL-6R) and, optionally, at least one additional therapeutic agent such as a disease modifying antirheumatic drug (DMARD).

### Anti-hIL-6R Antibodies

The present disclosure includes methods that comprise administering to a patient a human antibody, or an antigen-binding fragment thereof, that binds specifically to hIL-6R. As used herein, the term "hIL-6R" means a human cytokine receptor that specifically binds human interleukin-6 (IL-6). In certain aspects, the antibody that is administered to the patient binds specifically to the extracellular domain of hIL-6R. The extracellular domain of hIL-6R is shown in the amino acid sequence of SEQ ID NO:1.

Unless specifically indicated otherwise, the term "antibody," as used herein, shall be understood to encompass antibody molecules comprising two immunoglobulin heavy chains and two immunoglobulin light chains (*i.e.,* "full antibody molecules") as well as antigen-binding fragments thereof. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and (optionally) constant domains. Such DNA is known and/or is readily available from, *e.g.,* commercial sources, DNA libraries (including, *e.g.,* phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, *etc.*

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (*e.g.,* an isolated complementarity determining region (CDR)). Other engineered molecules, such as diabodies, triabodies, tetrabodies and minibodies, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric VH or VL domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H1}; (ii) V_{H}-C_{H2}; (iii) V_{H}-C_{H3}; (iv) V_{H}-C_{H1}-C_{H2}; (v) V_{H}-C_{H1}-C_{H2}-C_{H3}; (vi) V_{H}-C_{H2}-C_{H3}; (vii) V_{H}-C_{L}; (viii) V_{L}-C_{H1}; (ix) V_{L}-C_{H2}; (x) V_{L}-C_{H3}; (xi) V_{L}-C_{H1}-C_{H2}; (xii) V_{L}-C_{H1}-C_{H2}-CH3; (xiii) V_{L}-C_{H2}-C_{H3}; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.,* 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (*e.g*., by disulfide bond(s)).

The term "specifically binds," means that an antibody or antigen-binding fragment thereof forms a complex with an antigen that is relatively stable under physiologic conditions. Specific binding can be characterized by a dissociation constant of at least about 1x10⁻⁶ M or smaller. In other embodiments, the dissociation constant is at least about 1x10⁻¹ M, 1x10⁻⁸ M , or 1x10⁻⁹ M. Methods for determining whether two molecules specifically bind are well known in the art and include, for example, equilibrium dialysis, surface plasmon resonance, and the like.

As with full antibody molecules, antigen-binding fragments may be monospecific or multispecific (*e.g*., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present invention using routine techniques available in the art.

In specific embodiments, the antibody or antibody fragment for use in the method of the disclosure may be a multispecific antibody, which may be specific for different epitopes of one target polypeptide or may contain antigen-binding domains specific for epitopes of more than one target polypeptide. An exemplary bi-specific antibody format that can be used in the context of the present disclosure involves the use of a first immunoglobulin (Ig) C_{H3} domain and a second Ig C_{H3} domain, wherein the first and second Ig C_{H3} domains differ from one another by at least one amino acid, and wherein at least one amino acid difference reduces binding of the bispecific antibody to Protein A as compared to a bi-specific antibody lacking the amino acid difference. In one embodiment, the first Ig C_{H3} domain binds Protein A and the second Ig C_{H3} domain contains a mutation that reduces or abolishes Protein A binding such as an H95R modification (by IMGT exon numbering; H435R by EU numbering). The second C_{H3} may further comprise an Y96F modification (by IMGT; Y436F by EU). Further modifications that may be found within the second C_{H3} include: D16E, L18M, N44S, K52N, V57M, and V82I (by IMGT; D356E, L358M, N384S, K392N, V397M, and V422I by EU) in the case of IgG1 antibodies; N44S, K52N, and V82I (IMGT; N384S, K392N, and V422I by EU) in the case of IgG2 antibodies; and Q15R, N44S, K52N, V57M, R69K, E79Q, and V82I (by IMGT; Q355R, N384S, K392N, V397M, R409K, E419Q, and V422I by EU) in the case of IgG4 antibodies. Variations on the bi-specific antibody format described above are contemplated within the scope of the present invention.

A "neutralizing" or "blocking" antibody, as used herein, is intended to refer to an antibody whose binding to hIL-6R results in inhibition of the biological activity of hIL-6. This inhibition of the biological activity of hIL-6 can be assessed by measuring one or more indicators of hIL-6 biological activity known to the art, such as hIL-6-induced cellular activation and hIL-6 binding to hIL-6R (see examples below).

The fully-human anti-IL-6R antibodies disclosed herein may comprise one or more amino acid substitutions, insertions and/or deletions in the framework and/or CDR regions of the heavy and light chain variable domains as compared to the corresponding germline sequences. Such mutations can be readily ascertained by comparing the amino acid sequences disclosed herein to germline sequences available from, for example, public antibody sequence databases. The present disclosure includes antibodies, and antigen-binding fragments thereof, which are derived from any of the amino acid sequences disclosed herein, wherein one or more amino acids within one or more framework and/or CDR regions are back-mutated to the corresponding germline residue(s) or to a conservative amino acid substitution (natural or non-natural) of the corresponding germline residue(s) (such sequence changes are referred to herein as "germline back-mutations"). A person of ordinary skill in the art, starting with the heavy and light chain variable region sequences disclosed herein, can easily produce numerous antibodies and antigen-binding fragments which comprise one or more individual germline back-mutations or combinations thereof. In certain aspects, all of the framework and/or CDR residues within the VH and/or VL domains are mutated back to the germline sequence. In other aspects, only certain residues are mutated back to the germline sequence, *e.g*., only the mutated residues found within the first 8 amino acids of FR1 or within the last 8 amino acids of FR4, or only the mutated residues found within CDR1, CDR2 or CDR3. Furthermore, the antibodies of the present disclosure may contain any combination of two or more germline back-mutations within the framework and/or CDR regions, *i.e.,* wherein certain individual residues are mutated back to the germline sequence while certain other residues that differ from the germline sequence are maintained. Once obtained, antibodies and antigen-binding fragments that contain one or more germline back-mutations can be easily tested for one or more desired property such as, improved binding specificity, increased binding affinity, improved or enhanced antagonistic or agonistic biological properties (as the case may be), reduced immunogenicity, *etc.* Antibodies and antigen-binding fragments obtained in this general manner are encompassed within the present invention.

The term "epitope" refers to an antigenic determinant that interacts with a specific antigen binding site in the variable region of an antibody molecule known as a paratope. A single antigen may have more than one epitope. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sufonyl groups on the antigen.

The anti-hIL-6R can be sarilumab (SAR153191). Sarilumab is defined as an antibody comprising the heavy chain variable region of SEQ ID NO:2 and the light chain variable region of SEQ ID NO:3.

### DMARDs

Disease modifying antirheumatic drugs (DMARDs) include methotrexate, sulfasalazine, hydroxychloroquine and leflunomide. According to the compositions and methods of the disclosure, DMARDs can be administered as follows. Methotrexate can be administered from 10 to 25 mg per week orally or intramuscularly. In another embodiment, methotrexate is administered from 6 to 25 mg/week orally or intramuscularly for patients who are from the Asia-Pacific region or who are descended from people who are from the Asia-Pacific region. The Asia-Pacific region includes Taiwan, South Korea, Malaysia, Philippines, Thailand and India. In certain embodiments, methotrexate is administered at between 6 and 12, 10 and 15, 15 and 20 and 20 and 25 mg per week. In other embodiments, methotrexate is administered at 6, 7, 8, 9, 10, 11, 12, 13, 14, 15, 16, 17, 18, 19, 20, 21, 22, 23, 24 or 25 mg per week. Leflunomide can be administered from 10 to 20 mg orally daily. In certain embodiments, leflunomide can be administered at between 10 and 12, 12 and 15, 15 and 17 and 18 and 20 mg per day. In other embodiments, leflunomide is administered at 10, 11, 12, 13, 14, 15, 16, 17, 18, 19 or 20 mg per day. Sulfasalazine can be administered from 1000 to 3000 mg orally daily. In certain embodiments, sulfasalazine can be administered at between 1000 and 1400, 1400 and 1800, 1800 and 2200, 2200 and 2600, and 2600 and 3000 mg per day. In other embodiments, sulfasalazine is administered at 1000, 1100, 1200, 1300, 1400, 1500, 1600, 1700, 1800, 1900, 2000, 2100, 2200, 2300, 2400, 2500, 2600, 2700, 2800, 2900 or 3000 mg per day. Hydroxychloroquine can be administered from 200 to 400 mg orally daily. In certain embodiments, hydroxychloroquine can be administered at between 200 and 240, 240 and 280, 280 and 320, 320 and 360 and 360 and 400 per day. In other embodiments, hydroxychloroquine can be administered at 200, 210, 220, 230, 240, 250, 260, 270, 280, 290, 300, 310, 320, 330, 340, 350, 360, 370, 380, 390 or 400 mg per day.

### Therapeutic Administration and Formulations

The methods described herein comprise administering a therapeutically effective amount of an anti-hIL-6R antibody and, optionally, a DMARD to a subject. As used herein, the phrase "therapeutically effective amount" means a dose of the therapeutic that results in a detectable improvement in one or more symptoms associated with rheumatoid arthritis or which causes a biological effect (*e.g.,* a decrease in the level of a particular biomarker) that is correlated with the underlying pathologic mechanism(s) giving rise to the condition or symptom(s) of rheumatoid arthritis. For example, a dose of anti-hIL-6R antibody which causes an improvement in any of the following symptoms or conditions is deemed a "therapeutically effective amount": chronic disease anemia, fever, depression, fatigue, rheumatoid nodules, vasculitis, neuropathy, scleritis, pericarditis, Felty's syndrome and/or joint destruction.

A detectable improvement can also be detected using the American College of Rheumatism (ACR) rheumatoid arthritis classification criteria. For example a 20% (ACR20), 50% (ACR50) or 70% (ACR70) improvement from baseline can be used to show detectable improvement.

The disease activity score (DAS28) can be used to show detectable improvement. DAS28 is a composite score of tender joints count based on 28 joints, a swollen joints count based on 28 joints, a general health assessment and a marker of inflammation which can be assessed by measuring C-reactive protein (CRP) levels. The disease response can be presented using the European League against Rheumatism (EULAR) response criteria. A good response by this criteria is an improvement of greater than 1.2 in DAS28 score with a present score of greater than or equal to 3.2. A moderate response is an improvement of greater than 0.6 but less than or equal to 1.2 in DAS28 score and a present score of greater than 3.2. Non-response is an improvement of less than 0.6 in DAS28 score and a present score of greater than 5.1. DAS28 remission is a DAS28 score of less than 2.6.

Van der Heijde modified Total Sharp score (mTSS) can be used to show the degree of joint damage (also called structural damage). Typically, the progression of structural damage in a subject is measured by the change from Baseline (BL) of the Van der Heijde modified Total Sharp score (mTSS). Baseline (BL) is defined as the score obtained by the subject before being administered with sarilumab according to the invention. Change from baseline is defined as the difference existing between the score obtained by the subject at baseline and the score obtained by the subject after being administered with sarilumab according to the invention, typically measured after 24 or 52 weeks of treatment with sarilumab.By comparing the mTSS at baseline and after treatment with sarilumab, typically at 24 weeks or 52 weeks, it is possible to measure the progression of structural damage in the subject.

The mTSS methodology, which is standard in the field of Rheumatoid Arthritis, quantifies the extent of bone erosions for 44 joints and joint space narrowing for 42 joints, with higher scores representing greater damage. The van der Heijde mTSS at a time point is the sum of the scores from both the erosion score and the joint space narrowing score, for a maximum score of 448.

The joint erosion score is a summary of erosion severity in 32 joints of the hands and 12 joints of the feet. Each joint is scored, according to the surface area involved, from 0 to 5 for hand joints and 0 to 10 for the joints of the foot. The maximum erosion score (5 for the hand and 10 for the foot) indicates extensive loss of bone from more than one half of the articulating bone. A score of 0 in either the hand or foot indicates no erosion. The maximum erosion score is 280 (160 in the hands and 120 in the feet) for a time point.

The joint space narrowing (JSN) score summarizes the severity of JSN in 30 joints of the hands and 12 joints of the feet. Assessment of JSN for each hand (15 joints per hand) and foot (6 joints per foot), including subluxation, is scored from 0 to 4, with 0 indicating no/normal JSN and 4 indicating complete loss of joint space, bony ankylosis or luxation. Thus, the maximum JSN score is 168 at a time point.

Improvement of physical function of a subject suffering from rheumatoid arthritis, following administration of sarilumab according to the invention, is typically assessed by looking at a change from baseline (BL) in Health Assessment Questionnaire Disability Index (HAQ-DI) scores. Baseline is defined as the score obtained by the subject before being administered with sarilumab according to the invention. Change from baseline is defined as the difference existing between the score obtained by the subject at baseline and the score obtained by the subject after being administered with sarilumab according to the invention, typically measured after 16, 24 or 52 weeks of treatment with sarilumab.

The HAQ-DI is a standardized questionnaire developed at Stanford University for use in RA (available online). It is widely used throughout the world and has become a mandated outcome measure for clinical trials in rheumatoid arthritis.

The HAQ-DI, with the past week as the time frame, focuses on whether the respondent "is able to..." do the activity and covers eight categories in 20 items: dressing and grooming, arising, eating, walking, hygiene, reach, grip and activities, for which there are at least 2 questions by category. The four responses for the HAQ-DI questions are graded as follows: without any difficulty = 0; with some difficulty =1; with much difficulty = 2; and unable to do = 3. To calculate the Standard HAQ-DI Score (With Aids/Devices), there are three steps
1. Sum the 8 category scores by using the highest sub-category score from each category.
   - For example, in the category ARISING there are three sub-category items. A patient responds with a 1, 2, and 0, respectively; the category score is 2.
2. Adjust for use of aids/devices and/or help from another person when indicated.
   - Adjust the score for a category by increasing a zero or a one to a two.
   - If a patient's highest score for that sub-category is a two it remains a two, and if a three, it remains a three.
3. Divide the summed category scores by the number of categories answered (must be a minimum of 6) to obtain a HAQ-DI score of 0-3 (3=worst functioning).

A HAQ-DI score cannot be calculated validly when there are scores for less than six of the eight categories. HAQ-DI scoring ranges between 0 and 3. A high HAQ-DI score has been found to be a strong predictor of morbidity and mortality in RA.

In accordance with the methods of the present disclosure, a therapeutically effective amount of anti-hIL-6R antibody that is administered to the patient will vary depending upon the age and the size (*e.g.,* body weight or body surface area) of the patient as well as the route of administration and other factors well known to those of ordinary skill in the art. In certain embodiments, the dose of anti-hIL-6R antibody administered to the patient is from about 10 mg to about 500 mg. For example, the present invention includes methods wherein about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200, about 205 mg, about 210 mg, about 215 mg, about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, about 300, about 325 mg, about 350 mg, about 375 mg, about 400 mg, about 425 mg, about 450 mg, about 475 mg, about 500 mg, or more of anti-hIL-6R antibody is administered to the patient per week.

In one aspect, the hIL-6R antibody is administered at 100-150 mg per week. In another aspect, the hIL-6R antibody is administered at 100-200 mg per ever two weeks. In other aspects, the hIL-6R antibody is administered at about 100 or about 150 mg per week. In other aspects, the hIL-6R antibody is administered at about 100, 150 or 200 mg per every two weeks.

The amount of anti-hIL-6R antibody that is administered to the patient may be expressed in terms of milligrams of antibody per kilogram of patient body weight (*i.e.,* mg/kg). For example, the methods of the present disclosure include administering an anti-hIL-6R antibody to a patient at a daily dose of about 0.01 to about 100 mg/kg, about 0.1 to about 50 mg/kg, or about 1 to about 10 mg/kg of patient body weight.

The methods of the present disclosure include administering multiple doses of an anti-hIL-6R antibody to a patient over a specified time course. For example, the anti-hIL-6R antibody can be administered about 1 to 5 times per day, about 1 to 5 times per week, about 1 to 5 times per month or about 1 to 5 times per year. In certain embodiments, the methods of the invention include administering a first dose of anti-hIL-6R antibody to a patient at a first time point, followed by administering at least a second dose of anti-hIL-6R antibody to the patient at a second time point. The first and second doses, in certain embodiments, may contain the same amount of anti-hIL-6R antibody. For instance, the first and second doses may each contain about 10 mg to about 500 mg, about 20 mg to about 300 mg, about 100 mg to about 200 mg, or about 100 mg to about 150 mg of the antibody. The time between the first and second doses may be from about a few hours to several weeks. For example, the second time point (*i.e.,* the time when the second dose is administered) can be from about 1 hour to about 7 weeks after the first time point (*i.e.,* the time when the first dose is administered). According to certain exemplary embodiments of the present invention, the second time point can be about 1 hour, about 4 hours, about 6 hours, about 8 hours, about 10 hours, about 12 hours, about 24 hours, about 2 days, about 3 days, about 4 days, about 5 days, about 6 days, about 7 days, about 2 weeks, about 4 weeks, about 6 weeks, about 8 weeks, about 10 weeks, about 12 weeks, about 14 weeks or longer after the first time point. In certain embodiments, the second time point is about 1 week or about 2 weeks. Third and subsequent doses may be similarly administered throughout the course of treatment of the patient.

The disclosure provides methods of using therapeutic compositions comprising anti-IL-6R antibodies or antigen-binding fragments thereof and, optionally, one or more additional therapeutic agents, e.g., DMARDs. The therapeutic compositions of the disclosure will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose may vary depending upon the age and the weight of a subject to be administered, target disease, conditions, route of administration, and the like. Various delivery systems are known and can be used to administer the pharmaceutical composition of the disclosure, *e.g.,* encapsulation in liposomes, microparticles, microcapsules, receptor mediated endocytosis (see, *e.g.,* Wu et al. (1987) J. Biol. Chem. 262:4429-4432). Methods of introduction include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (*e.g.,* oral mucosa, rectal and intestinal mucosa, *etc.*) and may be administered together with other biologically active agents. Administration can be systemic or local. The hIL-6R antibody can be administered subcutaneously. The DMARD can be administered orally or intramuscularly.

The pharmaceutical composition can also be delivered in a vesicle, in particular a liposome (see Langer (1990) Science 249:1527-1533). In certain situations, the pharmaceutical composition can be delivered in a controlled release system, for example, with the use of a pump or polymeric materials. In another embodiment, a controlled release system can be placed in proximity of the composition's target, thus requiring only a fraction of the systemic dose.

The injectable preparations may include dosage forms for intravenous, subcutaneous, intracutaneous and intramuscular injections, local injection, drip infusions, *etc.* These injectable preparations may be prepared by methods publicly known. For example, the injectable preparations may be prepared, *e.g.,* by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, *etc.,* which may be used in combination with an appropriate solubilizing agent such as an alcohol (*e.g*., ethanol), a polyalcohol (*e.g.,* propylene glycol, polyethylene glycol), a nonionic surfactant [*e.g.,* polysorbate 80, HCO-50 (polyoxyethylene (50 mol) adduct of hydrogenated castor oil)], *etc.* As the oily medium, there are employed, *e.g.,* sesame oil, soybean oil, *etc.,* which may be used in combination with a solubilizing agent such as benzyl benzoate, benzyl alcohol, *etc.* The injection thus prepared can be filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, *etc.* The amount of the DMARD contained is generally about 5 to 3000 mg per dosage form in a oral unit dose depending on the specific DMARD used. The amount of the hIL-6R antibody contained is generally about 100 to 200 mg per subcutaneous dosage form.

In accordance with the methods disclosed herein, the anti-hIL-6R antibody (or pharmaceutical formulation comprising the antibody) can be administered to the patient using any acceptable device or mechanism. For example, the administration can be accomplished using a syringe and needle or with a reusable pen and/or autoinjector delivery device. The methods of the present disclosure include the use of numerous reusable pen and/or autoinjector delivery devices to administer an anti-hIL-6R antibody (or pharmaceutical formulation comprising the antibody). Examples of such devices include, but are not limited to AUTOPEN™ (Owen Mumford, Inc., Woodstock, UK), DISETRONIC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen and/or autoinjector delivery devices having applications in subcutaneous delivery of a pharmaceutical composition of the present disclosure include, but are not limited to the SOLOSTAR™ pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park, IL), to name only a few.

The use of a microinfusor to deliver an anti-hIL-6R antibody (or pharmaceutical formulation comprising the antibody) to a patient is also contemplated herein. As used herein, the term "microinfusor" means a subcutaneous delivery device designed to slowly administer large volumes (*e.g*., up to about 2.5 mL or more) of a therapeutic formulation over a prolonged period of time (*e.g*., about 10, 15, 20, 25, 30 or more minutes). See, *e.g.,* U.S. 6,629,949; US 6,659,982; and Meehan et al., J. Controlled Release 46:107-116 (1996). Microinfusors are particularly useful for the delivery of large doses of therapeutic proteins contained within high concentration (*e.g*., about 100, 125, 150, 175, 200 or more mg/mL) and/or viscous solutions.

### Combination Therapies

The present disclosure includes methods of treating rheumatoid arthritis which comprise administering to a patient in need of such treatment an anti-hIL-6R antibody. In certain embodiments, the anti-hIL-6 antibody is administered as a "monotherapy" or single therapeutic agent. In alternative embodiments, the anti-hIL-6 antibody is administered in combination with at least one additional therapeutic agent. Examples of additional therapeutic agents which can be administered in combination with an anti-hIL-6R antibody in the practice of the methods of the present disclosure include, but are not limited to DMARDs, and any other compound known to treat, prevent, or ameliorate rheumatoid arthritis in a human subject. Specific, non-limiting examples of additional therapeutic agents that may be administered in combination with an anti-hIL-6R antibody in the context of a method of the present disclosure include, but are not limited to methotrexate, sulfasalazine, hydroxychloroquine and leflunomide. In the present methods, the additional therapeutic agent(s) can be administered concurrently or sequentially with the anti-hIL-6R antibody. For example, for concurrent administration, a pharmaceutical formulation can be made which contains both an anti-hIL-6R antibody and at least one additional therapeutic agent. The amount of the additional therapeutic agent that is administered in combination with the anti-hIL-6R antibody in the practice of the methods of the present disclosure can be easily determined using routine methods known and readily available in the art.

The disclosure provides for pharmaceutical compositions comprising any of the following:
A composition comprising between 100 and 150 mg of sarilumab (SAR153191) and 10-25 mg of methotrexate.
A composition comprising between 100 and 200 mg of sarilumab (SAR153191) and 10-25 mg of methotrexate.
A composition comprising between 100 and 150 mg of sarilumab (SAR153191) and 6-25 mg of methotrexate.
A composition comprising between 100 and 200 mg of sarilumab (SAR153191) and 6-25 mg of methotrexate.
A composition comprising between 100 and 150 mg of sarilumab (SAR153191) and 10-20 mg of leflunomide.
A composition comprising between 100 and 200 mg of sarilumab (SAR153191) and 10-20 mg of leflunomide.
A composition comprising between 100 and 150 mg of sarilumab (SAR153191) and 1000-3000 mg of sulfasalazine.
A composition comprising between 100 and 200 mg of sarilumab (SAR153191) and 1000-3000 mg of sulfasalazine.
A composition comprising between 100 and 150 mg of sarilumab (SAR153191) and 200-400 mg of hydroxychloroquine.
A composition comprising between 100 and 200 mg of sarilumab (SAR153191) and 200-400 mg of hydroxychloroquine.

The disclosure of the invention provides for methods of improving symptoms associated with rheumatoid arthritis comprising any of the following:
A method comprising administering between 100 and 150 mg of sarilumab (SAR153191) and 10-25 mg of methotrexate per week to a subject in need thereof.
A method comprising administering between 100 and 200 mg of sarilumab (SAR153191) every two weeks and 10-25 mg of methotrexate per week to a subject in need thereof.
A method comprising administering between 100 and 150 mg of sarilumab (SAR153191) and 6-25 mg of methotrexate per week to a subject in need thereof.
A method comprising administering between 100 and 200 mg of sarilumab (SAR153191) every two weeks and 6-25 mg of methotrexate per week to a subject in need thereof.
A method comprising administering between 100 and 150 mg of sarilumab (SAR153191) per week and 10-20 mg of leflunomide per day to a subject in need thereof.
A method comprising administering between 100 and 200 mg of sarilumab (SAR153191) every two weeks and 10-20 mg of leflunomide per day to a subject in need thereof.
A method comprising administering between 100 and 150 mg of sarilumab (SAR153191) per week and 1000-3000 mg of sulfasalazine per day to a subject in need thereof.
A method comprising administering between 100 and 200 mg of sarilumab (SAR153191) every two weeks and 1000-3000 mg of sulfasalazine per day to a subject in need thereof.
A method comprising administering between 100 and 150 mg of sarilumab (SAR153191) per week and 200-400 mg of hydroxychloroquine per day to a subject in need thereof.
A method comprising administering between 100 and 200 mg of sarilumab (SAR153191) every two weeks and 200-400 mg of hydroxychloroquine per day to a subject in need thereof.

### Biomarkers

The present disclosure includes methods of treating rheumatoid arthritis by administering to a patient in need of such treatment a therapeutically effective amount of a human antibody or antibody binding fragment thereof which specifically binds to hIL-6R and a therapeutically effective amount of one or more DMARDs, wherein the level of one or more RA-associated biomarkers in the patient is modified (*e.g*., increased, decreased, *etc.,* as the case may be) following administration. In a related aspect, the present disclosure includes methods for decreasing an RA-associated biomarker in a patient by administering to the patient a therapeutically-effective amount of a human antibody or antigen-binding fragment thereof which specifically binds to hIL-6R and a therapeutically effective amount of one or more DMARDs.

Examples of RA-associated biomarkers include, but are not limited to, *e.g.,* high-sensitivity C-reactive protein (hsCRP), serum amyloid A (SAA), erythrocyte sedimentation rate (ESR), serum hepcidin, interleukin-6 (IL-6), and hemoglobin (Hb). As will be appreciated by a person of ordinary skill in the art, an increase or decrease in an RA-associated biomarker can be determined by comparing the level of the biomarker measured in the patient at a defined time point after administration of the anti-IL-6R antibody to the level of the biomarker measured in the patient prior to the administration (*i.e.,* the "baseline measurement"). The defined time point at which the biomarker can be measured can be, *e.g.,* at about 4 hours, 8 hours, 12 hours, 1 day, 2 days, 3 days, 4 days, 5 days, 6 days, 7 days, 8 days, 9 days, 10 days, 15 days, 20 days, 35 days, 40 days or more after administration of the anti-hIL-6R antibody.

According to certain embodiments of the present invention, a patient may exhibit a decrease in the level of one or more of hsCRP, SAA, ESR and/or hepcidin following administration of an anti-hIL-6R antibody to the patient. For example, at about week 12 following weekly administration of anti-hIL-6R antibody and one or more DMARDs the patient may exhibit one or more of the following: (i) a decrease in hsCRP by about 40%, 45%, 50%, 55%, 60%, 65%, 70%, 75%, 80%, 85%, 90%, 95% or more; (ii) a decrease in SAA by about 40%, 45%, 50%, 55%, 60%, 65%, 70%, 75%, 80%, 85%, 90%, 95% or more; (iii) a decrease in ESR by about 15%, 20%, 25%, 30%, 35%, 40%, 45%, 50%, 55% or more; and/or (iv) a decrease in hepcidin by about 30%, 35%, 40%, 45%, 50%, 55%, 60%, 65%, 70%, 75% or more.

According to certain other embodiments of the present invention, a patient may exhibit an increase in the level of one or more of Hb or IL-6 following administration of an anti-hIL-6R antibody and one or more DMARDs to the patient. For example, at about week 12 following weekly administration of anti-hIL-6R antibody and one or more DMARDs the patient may exhibit one or more of the following: (v) an increase in Hb by about 0.5%, 1.0%, 1.5%, 2.0%, 2.5%, 3.0%, 3.5%, 4.0%, 4.5%, 5.0%, 5.5%, 6.0% or more; and/or (vi) an increase in IL-6 by about 100%, 150%, 200%, 250%, 300%, 350%, 400%, 450%, 500%, 550%, 600%, 650%, 700%, 750%, 800% or more.

The present disclosure includes methods for determining whether a subject is a suitable patient for whom administration of an anti-hIL-6R antibody would be beneficial. For example, if an individual, prior to receiving an anti-hIL-6R antibody and/or one or more DMARDs, exhibits a level of an RA-associated biomarker which signifies the disease state, the individual is therefore identified as a suitable patient for whom administration of an anti-hIL-6R antibody would be beneficial. According to certain exemplary embodiments, an individual may be identified as a good candidate for anti-hIL-6R/DMARD therapy if the individual exhibits one or more of the following: (i) a level of hsCRP greater than about 4 mg/L (*e.g*., about 4.5 mg/L, about 5.0 mg/L, about 5.5 mg/L, about 6.0 mg/L, about 7.0 mg/L, about 10.0 mg/L, about 15.0 mg/L, about 20.0 mg/L, or more); (ii) a level of SAA greater than about 3800 ng/mL (*e.g*., about 4000 ng/mL, 4500 ng/mL, about 5000 ng/mL, about 5500 ng/mL, about 6000 ng/mL, about 10,000 ng/mL, about 20,000 ng/mL, about 25,000 ng/mL, about 30,000 ng/mL, about 35,000 ng/mL, about 40,000 ng/mL, about 45,000 ng/mL, or more); (iii) an ESR greater than about 15 mm/hr (*e.g*., about 16 mm/hr, about 17 mm/hr, about 18 mm/hr, about 19 mm/hr, about 20 mm/hr, about 21 mm/hr, about 22 mm/hr, about 25 mm/hr, about 30 mm/hr, about 35 mm/hr, about 40 mm/hr, about 45 mm/hr, about 50 mm/hr, or more); and/or (iv) a level of hepcidin greater than about 60 ng/mL (*e.g*., about 62 ng/mL, about 64 ng/mL, about 68 ng/mL, about 70 ng/mL, about 72 ng/mL, about 74 ng/mL, about 76 ng/mL, about 78 ng/mL, about 80 ng/mL, about 82 ng/mL, about 84 ng/mL, about 85 ng/mL, about 90 ng/mL, about 95 ng/mL, about 100 ng/mL, about 105 ng/mL, or more). Additional criteria, such as other clinical indicators of RA, may be used in combination with any of the foregoing RA-associated biomarkers to identify an individual as a suitable candidate for anti-hIL-6R therapy.

### Patient Population

In certain embodiments, the methods and compositions described herein are administered to specific patient populations. These populations include patients that have previously been treated for rheumatoid arthritis with treatment regimens other than the combination of an anti-hIL-6R antibody and one or more DMARDs. In other embodiments, sarilumab is administered to a patient who has previously been ineffectively treated with a DMARD and an anti-hIL-6R antibody other than sarilumab. These treatment regimens include anti-TNF-α therapy, e.g., biologic anti-TNF-α treatment regimens. Biologic anti-TNF-α antagonists include etanercept, infliximab, adalimumab, golimumab and certolizumab pegol. These treatment regimens also include DMARD therapy in the absence of anti-hIL-6R antibody.

DMARDs used in this therapy include methotrexate, sulfasalazine, hydroxychloroquine and leflunomide. The DMARDs may be administered alone or in combination with another therapy that is not an anti-hIL-6R antibody, e.g., Sarilumab. In a specific embodiment, the previous treatment regimen was methotrexate. In another embodiment, treatment with methotrexate is maintained in patient treated with an anti-hIL-6R antibody. In certain embodiments, the patient has previously been administered both anti-TNF-α and DMARD therapies. The therapies may be performed sequentially in any order or simultaneously. In certain embodiments, these therapies have been received by the patient within 2 years prior to receiving the combination of an anti-hIL-6R antibody and one or more DMARDs. In other embodiments, these therapies have been received within 1, 2, 3, 4, 5, 6, 7, 8, 9 or 10 years prior to receiving the combination of an anti-hIL-6R antibody and one or more DMARDs.

In certain aspects, the methods and compositions described herein are administered to specific patient populations that have received one or more of the treatment regimens described above wherein these treatments have not been effective. As used herein, a treatment has not been effective when the treatment (e.g., a dose of anti-TNF-a and/or a DMARD) does not result in a detectable improvement in one or more symptoms associated with rheumatoid arthritis or which does not cause a biological effect (*e.g.,* a decrease in the level of a particular biomarker) that is correlated with the underlying pathologic mechanism(s) giving rise to the condition or symptom(s) of rheumatoid arthritis. For example, a treatment which does not cause an improvement in any of the following symptoms or conditions is deemed ineffective: chronic disease anemia, fever, depression, fatigue, rheumatoid nodules, vasculitis, neuropathy, scleritis, pericarditis, Felty's syndrome and/or joint destruction.

A detectable improvement can also be detected using the American College of Rheumatism (ACR) rheumatoid arthritis classification criteria. For example a 20% (ACR20), 50% (ACR50) or 70% (ACR70) improvement from baseline can be used to show detectable improvement. Conversely, the ACR classification criteria may be used to select patients who have previously been ineffectively treated prior to treatment with anti-IL6R therapy. For example, a patient may be ineffectively treated if they fail to exhibit at least a 10% detectable improvement (e.g., a 10%, 20%, 50% or 70% improvement) according to ACR criteria.

In other embodiments, the disease activity score (DAS28) can be used to show detectable improvement or, conversely, ineffective treatment. DAS28 is a composite score of tender joints count based on 28 joints, a swollen joints count based on 28 joints, a general health assessment and a marker of inflammation which can be assessed by measuring C-reactive protein (CRP) levels. The disease response can be presented using the European League against Rheumatism (EULAR) response criteria. A good response by this criteria is an improvement of greater than 1.2 in DAS28 score with a present score of greater than or equal to 3.2. A moderate response is an improvement of greater than 0.6 but less than or equal to 1.2 in DAS28 score and a present score of greater than 3.2. Non-response is an improvement of less than 0.6 in DAS28 score and a present score of greater than 5.1. DAS28 remission is a DAS28 score of less than 2.6. A detectable improvement can also be shown by measuring an improvement in any of the components of the DAS28 score.

In other exemplary embodiments, a treatment has not been effective when a patient still presents an "active disease" after treatment. For example, patients present an "active disease" when they exhibit at least 8 of 68 tender joints and 6 of 66 swollen joints, and/or high sensitivity C-reactive protein (hs-CRP) >10 mg/L (>1.0 mg/dL). In a specific embodiment, sarilumab is administered to a patient who has previously been ineffectively treated with methotrexate (MTX). In such an example, patients may have received continuous treatment with MTX 10 to 25 mg/week (or per local labeling requirements if the dose range differs) for at least 12 weeks and on a stable dose of MTX for a minimum of 8 weeks and still present a moderate-to-severely active RA, defined as: (i) at least 8 of 68 tender joints and 6 of 66 swollen joints, and (ii) high sensitivity C-reactive protein (hs-CRP) >10 mg/L (>1.0 mg/dL).

In one embodiment, the patient population comprises subjects that have not received a biologic agent within the last three months. In certain embodiments, a biologic agent is a protein. According to other embodiments, the protein is an antibody or a fragment thereof. According to other embodiments, the protein is a cytokine or a fragment or derivative thereof. In other embodiments, the protein is recombinant. In other embodiments, the biologic agent is a vaccine, blood, blood component, allergenic, somatic cell, gene therapy or biological tissue. Biologics include blood factors, thrombolytic agents, hormones, hematopoietic growth factors, interferons, interleukin based products, vaccines and monoclonal antibodies. In certain embodiments, the biologic agent is abatacept. In other embodiments, the biologic agent is adalimumab. In other embodiments, biologic agents non-exhaustively include: TNFα blockers such as infliximab (Remicade®), adalimumab (Humira®), golimumab (Simponi®), etanercept (Enbrel®), certolizumab (Cimzia®); IL-1 blockers such as anakinra (Kineret®), anti-CD20 antibodies such as rituximab (Rituxan®), T cell costimulation blocker such as abatacept (Orencia®), and anti-IL6 antibodies such as sirukumab, clazakizumab or olokizumab.

According to other embodiments, the biologic is a biologic medical product. A biologic medical product is a medical product that is manufactured or extracted from biological sources. In certain embodiments, biologics are defined as excluding biological products that are consumed by animals for nutrition. In these embodiments, biologics must have a specific therapeutic consequence to the subject population beyond nutrition.

In another embodiment, the patient population comprises subjects that have received treatment for methotrexate for at least 6 weeks. In other embodiments, the subjects have received methotrexate for at least 1, 2, 3, 4, 5, 6, 7, 8, 9, 10, 11, 12, 13, 14, 15, 16, 17, 18, 19, 20, 21, 22, 23, 24, 25 or 26 weeks. In more specific embodiments, the subjects have received methotrexate but have not received other DMARDs for at least 6 weeks. In other embodiments, the subjects have not received a DMARD other than methotrexate for at least 1, 2, 3, 4, 5, 6, 7, 8, 9, 10, 11, 12, 13, 14, 15, 16, 17, 18, 19, 20, 21, 22, 23, 24, 25 or 26 weeks. DMARDs other than methotrexate include leflunomide, sulfasalazine and hydroxychloroquine.

In another embodiment, the patient population comprises subjects that are not suffering from an autoimmune disease other than rheumatoid arthritis. In certain embodiments, the autoimmune disease other than arthritis is one or more pathologies selected from the group consisting of Acute disseminated encephalomyelitis (ADEM), Addison's disease, Agammaglobulinemia, Alopecia areata, Amyotrophic lateral sclerosis (Also Lou Gehrig's disease; Motor Neuron Disease), Ankylosing Spondylitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative, Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Balo disease/Balo concentric sclerosis, Behçet's disease, Berger's disease, Bickerstaff's encephalitis, Blau syndrome, Bullous pemphigoid, Cancer, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, Crohn's disease, Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic, Eosinophilic pneumonia, Epidermolysis bullosa acquisita, Erythema nodosum, Erythroblastosis fetalis, Essential mixed cryoglobulinemia, Evan's syndrome, Fibrodysplasia ossificans progressiva, Fibrosing alveolitis, Gastritis, Gastrointestinal pemphigoid, Glomerulonephritis, Goodpasture's syndrome, Graves' disease, Guillain-Barre syndrome (GBS), Hashimoto's encephalopathy, Hashimoto's thyroiditis, Henoch-Schonlein purpura, Herpes gestationis, Hidradenitis suppurativa, Hughes-Stovin syndrome, Hypogammaglobulinemia, Idiopathic inflammatory demyelinating diseases, Idiopathic pulmonary fibrosis, Idiopathic thrombocytopenic purpura, IgA nephropathy, Inclusion body myositis, Chronic inflammatory demyelinating polyneuropathy, Interstitial cystitis, Juvenile idiopathic arthritis, Kawasaki's disease, Lambert-Eaton myasthenic syndrome, Leukocytoclastic vasculitis, Lichen planus, Lichen sclerosus, Linear IgA disease (LAD), Lupoid hepatitis, Lupus erythematosus, Majeed syndrome, Ménière's disease, Microscopic polyangiitis, Miller-Fisher syndrome, Mixed connective tissue disease, Morphea, Mucha-Habermann disease, Multiple sclerosis, Myasthenia gravis, Microscopic colitis, Myositis, Narcolepsy, Neuromyelitis optica, Neuromyotonia, Occular cicatricial pemphigoid, Opsoclonus myoclonus syndrome, Ord's thyroiditis, Palindromic rheumatism, PANDAS (pediatric autoimmune neuropsychiatric disorders associated with streptococcus), Paraneoplastic cerebellar degeneration, Paroxysmal nocturnal hemoglobinuria, Parry Romberg syndrome, Parsonage-Turner syndrome,Pars planitis, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatic fever, Sarcoidosis, Schizophrenia, Schmidt syndrome, Schnitzler syndrome, Scleritis, Scleroderma, Serum Sickness, Sjögren's syndrome, Spondyloarthropathy, Still's disease, Stiff person syndrome, Subacute bacterial endocarditis, Susac's syndrome, Sweet's syndrome, Sydenham chorea, Sympathetic ophthalmia, Systemic lupus erythematosus, Takayasu's arteritis, Temporal arteritis, Thrombocytopenia, Tolosa-Hunt syndrome, Transverse myelitis, Ulcerative colitis, Undifferentiated connective tissue disease, Undifferentiated spondyloarthropathy, Urticarial vasculitis, Vasculitis, Vitiligo and Wegener's granulomatosis

In another embodiment, the patient population comprises subjects that have not received parenteral or intra-articular administration of glucocorticoids for four weeks. In other embodiments, the subjects have not received parenteral or intra-articular administration of glucocorticoids for at least 1, 2, 3, 4, 5, 6, 7, 8, 9, 10, 11, 12, 13, 14, 15, 16, 17, 18, 19, 20, 21, 22, 23, 24, 25 or 26 weeks. In certain embodiments, the glucocorticoids include one or more selected from the group consisting of cortisol, cortisone, prednisone, prednisolone, methylprednisolone, dexamethasone, betamethasone, fludrocortisone, deoxycorticosterone acetate and aldosterone.

### EXAMPLES

### Example 1. Combination of Sarilumab and Methotrexate is Effective in Treatment of Rheumatoid Arthritis in Patients where Methotrexate Treatment is Ineffective.

A worldwide, double-blind, placebo-controlled, randomized study was performed in patients with rheumatoid arthritis with an inadequate response to methotrexate (MTX). Patients who were included in the study had the following criteria. Patients needed to have active disease defined as: at least 6 of 66 swollen joints and 8 of 68 tender joints and; hs-CRP > 6 mg/L. Patients also needed to have had continuous treatment with methotrexate (MTX) - 10 to 25 mg/wk (or 6 to 25 mg/wk for patients within Asia-Pacific region for 12 weeks.

The study included two parts. The first part (Part A) of the study was a 12-week, 6-arm dose-ranging part intended to select the two best dose regimens based on efficacy (reduction in signs and symptoms) and safety. The second part (Part B) of the study was a 52-week part to confirm the efficacy and safety of these two selected dose regimens on reduction in signs and symptoms, inhibition of progression of structural damage, improvement in physical function, and induction of major clinical response.

The operationally seamless design nature of this study resided in the fact that Part B started to test patients just after the last patient was randomized in Part A without waiting for the dose selection based on its results. Thus part B patients belong to 2 distinct cohorts according to the time of their enrollment:
Cohort 1 of patients was randomized before the dose selection: these patients were randomized into six arms (as the ones of Part A). After dose selection, the patients randomized in the two selected doses and the placebo regimens continued the 52-week trial but those randomized in the three other arms discontinued from the present study but proposed to join an open label extension (see LTS11210).
Cohort 2 of patients randomized after the dose selection: these patients were randomized into three arms, the two selected ones and placebo.

### Part A

Patients were assessed at a screening visit for confirmation of the diagnosis, disease activity, eligibility to the study and verification of concomitant therapy. Complete examination and laboratory tests including hematology, chemistry profile, lipid profile, liver enzymes and acute phase reactants, HbA1c, hepatitis B and C and serum pregnancy test for women of childbearing potential were performed. An ECG evaluation was also performed. A PPD test and QuantiFERON were performed to exclude any tuberculosis as well as a chest X-ray (if a documented negative X-ray performed in the last 3 months is not available).

After confirmation of eligibility, patients were randomized in a balanced manner, in this international multi-center, double-blind, parallel group placebo-controlled, 12-week study treatment of six arms of SAR153191 or placebo given subcutaneously weekly with MTX cotherapy:

| **Arm** | **Regimen** | **No. Patients** |
|---|---|---|
| Arm 1 | Sarilumab SC 100mg every week (qw) + MTX | 50 |
| Arm 2 | Sarilumab SC 150mg every week (qw) + MTX | 50 |
| Arm 3 | Sarilumab SC 100mg every 2 weeks (q2w) + MTX | 50 |
| Arm 4 | Sarilumab SC 150mg every 2 weeks (q2w) + MTX | 50 |
| Arm 5 | Sarilumab SC 200mg every 2 weeks (q2w) + MTX | 50 |
| Arm 6 | Placebo SC + MTX | 50 |

Methothrexate was administered for each patient as it had been before the study. This was at 10 to 25 mg/wk, or 6 to 25 mg/wk for patients within Asia-Pacific region; Taiwan, South Korea, Malaysia, Philippines, Thailand, and India.

During the first visit, patients were reminded of the list of prohibited medications, and that they should continue taking MTX at their current stable dose until the end of the study with folic acid as per local recommendation to prevent MTX toxicity. The patients were trained to prepare and self administer the IMP and were reminded to have injection strictly 7 days apart. At dosing time points occurring outside site visits, SAR153191 was injected by the patient himself, by a trained professional caregiver or by a trained qualified person.

Patients had six additional visits at weeks 2, 4, 6, 8, 10, and 12. Efficacy assessment and laboratory test including hematology, chemistry profile, lipid profile, liver enzymes and acute phase reactants were assessed throughout the study to allow calculation of the main efficacy scores, and follow up of safety aspects. At randomization visit and at Week 2, 4, 8, and 12, a complete joint examination for tender joint count and swollen joint count was performed by an assessor independent from the Investigator and the patient's data, in order to calculate the ACR score (primary endpoint). In order to maintain the blind, the Investigator, the Sponsor and the patient will be blind to CRP and serum IL6 levels during the study.

A close monitoring of adverse events including potential infections assessed in part by monitoring of body temperature was performed at every visit. Presence of tuberculosis was checked through specific patient assessment (check for any signs or symptoms, or contact with active TB). Neurological abnormalities (history and physical examination) or autoimmune diatheses (ANA, ds-DNA antibodies) were tested at baseline and end of treatment visit.

Specific blood and urine samples were taken during the study to test potential biomarkers that may be predictive of disease response or adverse events. These included a single sample for DNA (after the patient has signed a specific informed consent form) and several samples obtained sequentially throughout the study for RNA expression-profiling and protein biomarker analyses. Samples were also collected at appropriate time points for pharmacokinetic parameters and antibody to SAR153191.

Patients prematurely discontinued were evaluated at an end of treatment visit with complete clinical and laboratory evaluation. They were considered as nonresponders with regard to the ACR score.

At the end of treatment visit, all patients were scheduled to complete a Post Treatment Follow-up Visit. Patients who had completed the treatment period were proposed to enter an open-label long-term safety extension study with SAR153191.

### Results

Human patients treated with sarilumab (REGN88/SAR153191) in combination with the standard RA treatment, methotrexate (MTX), achieved a significant and clinically meaningful improvement in signs and symptoms of moderate-to-severe rheumatoid arthritis (RA) compared to patients treated with MTX alone. The 306-patient, dose-ranging, multinational, randomized, multi-arm, double-blind, placebo-controlled study was performed that compared five different dose regimens of sarilumab in combination with MTX to placebo plus MTX. The primary endpoint of the study was the proportion of patients achieving at least a 20% improvement in RA symptoms (ACR20) after 12 weeks.

A dose response was observed in patients receiving sarilumab in combination with MTX. An ACR20 response after 12 weeks was seen in 49.0% of patients receiving the lowest sarilumab dose regimen and 72.0% of patients receiving the highest dose regimen compared to 46.2% of patients receiving placebo and MTX (see table below, asterisk (*) indicates significance versus placebo by Cochran-Mantel-Haenszel test (p<0.05), correcting for multiplicity by Hommel's procedure).

| **Regimen** | **No. Patients** | **ACR20 (%)** |
|---|---|---|
| Sarilumab SC 100mg every week (qw) + MTX | 50 | 62 |
| Sarilumab SC 150mg every week (qw) + MTX | 50 | 72* |
| Sarilumab SC 100mg every 2 weeks (q2w) + MTX | 51 | 49 |
| Sarilumab SC 150mg every 2 weeks (q2w) + MTX | 51 | 67 |
| Sarilumab SC 200mg every 2 weeks (q2w) + MTX | 52 | 65 |
| Placebo SC + MTX | 52 | 46 |

The most common adverse events (>5%) reported more frequently in active-treatment arms included infections (non-serious), neutropenia, and liver-function test abnormalities. The types and frequencies of adverse events were consistent with those previously reported with IL-6 inhibition. The incidence of serious adverse events among the five sarilumab treatment groups and the placebo group were comparable.

Sarilumab also demonstrated significant benefit compared to placebo in secondary endpoints, including ACR 50, ACR 70, and DAS 28 scores, additional measures of clinical activity used in RA trials. More specifically:
- An ACR50 response after 12 weeks was seen in 22% of patients receiving the lowest sarilumab dose regimen and 30% of patients receiving the highest dose regimen compared to 15% of patients receiving placebo and MTX (see table below, double asterisk (**) indicates statistical significance versus placebo (p<0.01, post hoc adjusted for multiplicity).

| **Regimen** | **No. Patients** | **ACR50 (%)** |
|---|---|---|
| Sarilumab SC 100mg every week (qw) + MTX | 50 | 40** |
| Sarilumab SC 150mg every week (qw) + MTX | 50 | 30 |
| Sarilumab SC 100mg every 2 weeks (q2w) + MTX | 51 | 22 |
| Sarilumab SC 150mg every 2 weeks (q2w) + MTX | 51 | 35 |
| Sarilumab SC 200mg every 2 weeks (q2w) + MTX | 52 | 40** |
| Placebo SC + MTX | 52 | 15 |

- The ACR70 response was also significantly higher in the 200 mg q2w group versus placebo. An ACR70 response after 12 weeks was seen in 16% of patients receiving the highest dose regimen compared to 2% of patients receiving placebo and MTX ((see table below, double asterisk (**) indicates statistical significance versus placebo (p<0.01, post hoc adjusted for multiplicity).

| **Regimen** | **No. Patients** | **ACR70 (%)** |
|---|---|---|
| Sarilumab SC 100mg every week (qw) + MTX | 50 | 16 |
| Sarilumab SC 150mg every week (qw) + MTX | 50 | 16 |
| Sarilumab SC 100mg every 2 weeks (q2w) + MTX | 51 | 6 |
| Sarilumab SC 150mg every 2 weeks (q2w) + MTX | 51 | 12 |
| Sarilumab SC 200mg every 2 weeks (q2w) + MTX | 52 | 17** |
| Placebo SC + MTX | 52 | 2 |

- A statistically-significant improvement in 28 score was seen after 12 weeks in all but the lowest dose regimens (see table below, which depicts the clinical trial results regarding the secondary endpoint of improved DAS28 score or DAS28 remission after 12 weeks in patients receiving one of 5 treatment arms of sarilumab / MTX cotherapy in comparison with placebo. LS (least squares), SE (standard error). †p<0.01; ††p<0.001; p<0.01 is considered statistically significant versus placebo after post hoc adjustment for multiplicity).

| **Regimen** | **No. Patients** | **Change in DAS28 at week 12, LS mean change from baseline (SE)** | **DAS28 remission at week 12, n(%)** |
|---|---|---|---|
| Sarilumab SC 100mg every week (qw) + MTX | 49 | -2.4 | 10 |
| | | (0.2) †† | (20.4) |
| Sarilumab SC 150mg every week (qw) + MTX | 50 | -2.5 | 15 |
| | | (0.2) †† | (30.0) †† |
| Sarilumab SC 100mg every 2 weeks (q2w) + MTX | 51 | -1.4 | 4 |
| | | (0.2) | (7.8) |
| Sarilumab SC 150mg every 2 weeks (q2w) + MTX | 51 | -2.3 | 10 |
| | | (0.2) †† | (19.6) |
| Sarilumab SC 200mg every 2 weeks (q2w) + MTX | 50 | -2.5 | 13 |
| | | (0.2) †† | (26.0) † |
| Placebo SC + MTX | 52 | -1.2 | 2 |
| | | (0.2) | (3.8) |

At the four highest dosage regimens, a decreased DAS score of at least 2.0 (versus baseline) was observed at the four highest dosage regimens after 12 week. Moreover, DAS remissions (patients with a DAS28 score of <2.6) were significantly higher in the 200 mg q2w and 150 mg qw treatment groups relative to placebo after 12 weeks.
- Significant improvements in the ACR criteria of Swollen Joint Count (SJC) and Tender Joint Count (TJC). For example, TJC was decreased by 10 in all dosage regimens after 12 weeks, while SJC was decreased by 8 at the four highest treatment dosages. By contrast, the placebo group exhibited a decreased TJC and SJC of 7 and 9, respectively. Results are presented in table below (SJC = swollen joint count; TJC = tender joint count; after 12 weeks in patients receiving one of 5 treatment arms of sarilumab / MTX cotherapy in comparison with placebo):

| **Regimen** | **SJC** | **TJC** |
|---|---|---|
| Sarilumab SC 100mg every week (qw) + MTX | 10 | 14 |
| Sarilumab SC 150mg every week (qw) + MTX | 9 | 13 |
| Sarilumab SC 100mg every 2 weeks (q2w) + MTX | 6 | 12 |
| Sarilumab SC 150mg every 2 weeks (q2w) + MTX | 9 | 16 |
| Sarilumab SC 200mg every 2 weeks (q2w) + MTX | 10 | 15 |
| Placebo SC + MTX | 7 | 9 |

- Significant improvements in the Health Assessment Questionnaire Disability Index (HAQ-DI). For example, HAQ-DI scores decreased by at least 0.3 relative to baseline for all treatment groups, and at least 0.5 in the 150 mg 2qw and 200 mg 2qw treatment groups. Results are presented in the table below (HAQ-DI after 12 weeks in patients receiving one of 5 treatment arms of sarilumab / MTX cotherapy in comparison with placebo):

| **Regimen** | **HAQ-DI n.d**. |
|---|---|
| Sarilumab SC 100mg every week (qw) + MTX | -0.42 |
| Sarilumab SC 150mg every week (qw) + MTX | -0.45 |
| Sarilumab SC 100mg every 2 weeks (q2w) + MTX | -0.35 |
| Sarilumab SC 150mg every 2 weeks (q2w) + MTX | -0.62 |
| Sarilumab SC 200mg every 2 weeks (q2w) + MTX | -0.57 |
| Placebo SC + MTX | -0.26 |

- Significant improvements in Visual Acuity Score (VAS). For example, physician VAS decreased by at least 30 (relative to baseline) in the four highest dose regimens. Moreover, patient VAS and pain VAS decreased by at least 25 (relative to baseline) in the four highest dose regimens. Results are presented in table below (Visual Analog Scale (VAS) after 12 weeks in patients receiving one of 5 treatment arms of sarilumab / MTX cotherapy in comparison with placebo; VAS (mm) was evaluated in terms of patient VAS, physician VAS and pain VAS):

| **Regimen** | **Pain VAS** | **Physician VAS** | **Patient VAS** |
|---|---|---|---|
| Sarilumab SC 100mg every week (qw) + MTX | -29.19 | -35.20 | -30.22 |
| Sarilumab SC 150mg every week (qw) + MTX | -25.26 | -34.91 | -27.80 |
| Sarilumab SC 100mg every 2 weeks (q2w) + MTX | -21.02 | -28.85 | -20.12 |
| Sarilumab SC 150mg every 2 weeks (q2w) + MTX | -29.05 | -34.32 | -27.57 |
| Sarilumab SC 200mg every 2 weeks (q2w) + MTX | -32.46 | -39.66 | -31.66 |
| Placebo SC + MTX | -22.28 | -26.79 | -21.10 |

- Significant improvements in the level of C-reactive protein (CRP). For example, CRP levels decreased by at least 1mg/dL in all treatment groups. Moreover, CRP levels decreased by at least 2 mg/dL for all but the lowest dosage regimens. Results are presented in the table below (increase in C-reactive Protein (CRP) after 12 weeks in patients receiving one of 5 treatment arms of sarilumab / MTX cotherapy in comparison with placebo):

| **Regimen** | **CRP (mg/dL)** |
|---|---|
| Sarilumab SC 100mg every week (qw) + MTX | -2.50 |
| Sarilumab SC 150mg every week (qw) + MTX | -2.07 |
| Sarilumab SC 100mg every 2 weeks (q2w) + MTX | -1.02 |
| Sarilumab SC 150mg every 2 weeks (q2w) + MTX | -2.19 |
| Sarilumab SC 200mg every 2 weeks (q2w) + MTX | -2.19 |
| Placebo SC + MTX | -0.31 |

- An improved EULAR (European League Against Rheumatism) index at 12 weeks. For example, a "good response" according to the EULAR index was achieved in at least 18% of patients for all treatment groups. Indeed, a good response was observed in at least 30% of patients for all but the lowest dosage treatment group.

Results are presented in the table below (improvement in the EULAR (European League Against Rheumatism) index after 12 weeks in patients receiving one of 5 treatment arms of sarilumab / MTX cotherapy in comparison with placebo. ^{a}Cochran-Mantel-Haenszel test stratified by prior biologic use and region comparing nonresponders vs. responders (combined Good and Moderate Response). †p<0.01; ††p<0.001; p<0.01 is considered statistically significant versus placebo after post hoc adjustment for multiplicity):

| **Regimen** | **EULAR response at week 12, n (%)** | | |
|---|---|---|---|
| | **No response^{a}** | **Moderate response** | **Good response** |
| Sarilumab SC 100mg every week (qw) + MTX | 6(12%†) | 25(51%) | 18(37%) |
| Sarilumab SC 150mg every week (qw) + MTX | 8(16%†) | 21(42%) | 21(42%) |
| Sarilumab SC 100mg every 2 weeks (q2w) + MTX | 17(33%) | 25(49%) | 9(18%) |
| Sarilumab SC 150mg every 2 weeks (q2w) + MTX | 5(10%†) | 29(57%) | 17(33%) |
| Sarilumab SC 200mg every 2 weeks (q2w) + MTX | 7(14%†) | 22(44%) | 21(42%) |
| Placebo SC + MTX | 21(40%) | 27 (52%) | 4 (8%) |

These results provide evidence that IL-6R blockade with sarilumab represents a promising new anti-inflammatory investigational therapy for reducing RA disease symptoms.

### Part B

Patients were assessed at a screening visit for confirmation of the diagnosis disease activity, eligibility to the study and verification of concomitant therapy. The Investigator checked that the patient is either positive anticyclic citrullinated peptide antibody (CCP) or positive rheumatoid factor (RF) or that he/she had a confirmed bone erosion on an X-ray. If necessary, for patients who were both CCP and RF negative and had no X-ray, a centrally-reviewed screening X-ray was performed and considered also as the baseline X-ray assessment for the study.

Cohort 1: Patients randomized before the dose selection.

Recruitment in for the long term safety extension study started just after the last patient has been randomized in Part A. After confirmation of eligibility, patients were randomized, in a balanced manner stratified by prior biologic use and by regions, in an international, multi-center, double-blind, parallel group placebo-controlled, study treatment of 6 arms of SAR153191 (5 active dose regimens) or placebo given subcutaneously weekly with MTX cotherapy.

At the beginning of every patient visit for Cohort 1 patients, the Investigator checked through IVRS list that the patient is still "eligible" for the study, *i.e.,* that the patient was not to be discontinued because of randomization in a nonselected arm. Indeed, when the pivotal dose regimens were selected from Part A, only patients randomized in the corresponding arms or placebo were still be considered eligible for the study and will continue in the study for a total of 52 weeks. The other patients (randomized in the nonselected dose regimens) were considered no longer eligible by IVRS. The Investigator proposed these patients to participate in an open extension study with SAR153191 at the highest dose regimen available at the time the patient was enrolled.

The initial randomization remained blinded for all patients.

Cohort 2: Patients randomized after the dose selection - Pivotal Part.

At day 1, after confirmation of eligibility, patients were randomized, in a balanced manner stratified by prior biologic use and by regions, in an international, multi-center, double-blind, parallel group, placebo-controlled, study of 3 arms of SAR153191 (2 pivotal dose regimens) or placebo given subcutaneously with MTX cotherapy.

### Both Cohorts:

In either cohort, patients were evaluated at Week 2, at Week 4, and every 4 weeks until Week 28 and then every 8 weeks until Week 52 for efficacy and safety assessments and laboratory tests.

The same procedures as described in Part A were applied in Part B. In addition, an X-ray evaluation of the hands and feet joints was performed at baseline, Week 24 and Week 52. Radiographs de-identified of any patient information were sent to central readers for calculation of the Sharp score (a specific scoring system of joints destruction). Health economic assessments will be also added such as SF-36.

From Week 16, patients with lack of efficacy defined as less than 20% improvement from baseline in either swollen joints count (SJC) or tender joints count (TJC) for 2 consecutive visits, or any other clear lack of efficacy based on Investigator judgment were proposed to be rescued with open-label SAR153191 highest available dose at the time of transfer into the rescue treatment arm, and were continue in the study according to their planned visit schedule. Blood samples for laboratory analysis were taken two weeks after the switch for safety purpose. They were considered nonresponders for the primary endpoint (ACR20). These patients stayed in the study and continued all visits.

In selected countries, patients who met lack of efficacy criteria at Part B treatment Visit 7/Week 16, or thereafter, were permanently discontinued from treatment, and were not be eligible to participate in the open treatment rescue arm. Instead, the patients had a follow-up visit to evaluate safety 6 weeks after the End of Treatment visit.

For any patient who discontinued prematurely or who was prematurely rescued with open SAR153191, an additional X-ray evaluation was performed at the time of withdrawal or rescue, unless a study X-ray assessment had been performed within the preceding 3 months (a window of 3 months between 2 X-ray evaluations should be considered to avoid over X-ray exposure).

Patients completing Part B (including those in the open-label rescue arm) were proposed to be rolled into an open label extension study at the maximum dose regimen at the time of enrollment. All patients were scheduled to complete the Post Treatment Follow-up Visit. If the patient agreed to enter the SAR153191 open-label long-term extension study, and was confirmed to be eligible, the Post Treatment Follow-up Visit was not completed.

### Example 2. Combination of Sarilumab and DMARDs are Effective in Treatment of Rheumatoid Arthritis in Patients where TNF-α Antagonist and Methotrexate Treatment are Ineffective.

A worldwide, double-blind, placebo-controlled, randomized study was performed in patients with rheumatoid arthritis with an inadequate response to methotrexate (MTX) and at least one TNF-α antagonist. Patients who were included in the study had the following criteria. Patients had, in the opinion of the investigator, an inadequate response to at least one TNF-α antagonist, after being treated for at least 3 months in the last 2 years, or patients being intolerant to at least 1 TNF-α antagonist, resulting in discontinuation. TNF-α antagonists included etanercept, infliximab, adalimumab, golimumab and/or certolizumab pegol. Patients needed to have active disease defined as: at least 6 of 66 swollen joints and 8 of 68 tender joints and; hs-CRP ≥8 mg/L. Patients also needed to have had continuous treatment with one or a combination of DMARDs for at least 12 weeks prior to baseline and on a stable dose(s) for at least 6 weeks prior to screening. These DMARDs included methotrexate (MTX) - 10 to 25 mg/wk (or 6 to 25 mg/wk for patients within Asia-Pacific region; leflunomide (LEF) - 10 to 20 mg daily; sulfasalazine (SSZ) - 1000 to 3000 mg daily; or hydroxychloroquine (HCQ) - 200 to 400 mg daily.

**Table 1 - Groups and forms for both investigational medicinal product and noninvestigational medicinal product**

| **Group** | **Treatment** | **Sarilumab 150 mg** | **Sarilumab 200 mg** | **Placebo** | **Background medication as monotherapy or in combination** |
|---|---|---|---|---|---|
| I | BT + sarilumab every 2 weeks (q2w) | 1 SC injection | | -- | Including: - Methotrexate - 10 to 25 mg/wk (or 6 to 25 mg/wk for patients within Asia-Pacific region) with folic/folinic acid supplement |
| | | | | | - Leflunomide - 10 to 20 mg daily |
| | | | | | - Sulfasalazin - 1000 to 3000 mg daily |
| | | | | | - Hydroxychloroquin - 200 to 400 mg daily |
| II | BT + sarilumab q2w | -- | 1 SC injection | -- | Same as above |
| III | BT + placebo q2w | -- | -- | 1 SC injection | Same as above |
| From Week 12 patients with lack of efficacy defined as less than 20% improvement from baseline in both swollen joint count and tender joint count for 2 consecutive visits will be proposed to be rescued with open label sarilumab at the highest dose in the trial. These patients will continue the trial according to the schedule of visits. | | | | | |
| BT = background therapy; q2w = every other week; SC = subcutaneous | | | | | |

Subcutaneous administration will occur in the abdomen or thigh. Each dose will be self-administered (whenever possible), in a single injection. The SC injection sites can be alternated between the 4 quadrants of the abdomen (except the navel or waist area) or the thigh (front and side).

Patients and/or their nonprofessional caregivers will be trained to prepare and administer IMP at the start of the double-blind treatment period. This training must be documented in the patients' study file. The study coordinator or designee may administer the first injection comprising the initial dose as part of the training procedure on Day 1 (Visit 2). On days when the patient has a study visit, the IMP will be administered following clinic procedures and blood collection. For doses not given at the study site, diaries will be provided to record information pertaining to these injections; these diaries will be kept as source data in the patients' study file. If the patient is unable or unwilling to administer IMP, arrangements must be made for qualified site personnel and/or caregiver to administer IMP for the doses that are not scheduled to be given at the study site.

If the study visit is not performed at the site as scheduled, the dose will be administered by the patient and/or their caregiver(s) as scheduled.

Treatment will last for 24 weeks. From Week 12, patients with lack of efficacy defined as less than 20% improvement from baseline in both SJC and TJC for 2 consecutive visits will be proposed to be rescued with open label sarilumab at the highest dose in the trial. These patients will continue the trial according to the schedule of visits.

In this study, sarilumab is administered on top of DMARD therapy, considered as a background therapy. All patients should continue to receive continuous treatment with one or a combination of nonbiologic DMARD(s) as background therapy for at least 12 weeks prior to baseline and on a stable dose(s) for at least 6 weeks prior to screening:
- methotrexate (MTX) - 10 to 25 mg/wk (or 6 to 25 mg/wk for patients within Asia-Pacific region) with folic/folinic acid supplement
- leflunomide (LEF) - 10 to 20 mg daily
- sulfasalazin (SSZ) - 1000 to 3000 mg daily
- hydroxychloroquin (HCQ) - 200 to 400 mg daily

Each DMARD dose will be recorded throughout the study on the case report form. At any time, the DMARD dose can be reduced for safety or tolerability reason. Any change in dose and the start date of the new dose should be recorded on the e-CRF at every visit. DMARD(s) will not be dispensed or supplied by the Sponsor as an IMP.

All patients taking MTX will receive folic/folinic acid according to local recommendation in the country where the study is conducted. The dose, route and administration schedule of folic/folinic acid will be recorded with concomitant medications.

Sarilumab and matching placebo will be provided in identically matched glass prefilled syringes. Each prefilled syringe contains 1.14 mL of sarilumab (SAR153191) or matching placebo solution.

A list of treatment kit numbers will be generated. A randomization list will be generated by the interactive voice response system (IVRS). Both the randomization and treatment kit lists will be loaded into the IVRS.

The treatment kit numbers will be obtained by the Investigator at the time of patient randomization and subsequent patient scheduled visits via IVRS that will be available 24 hours a day.

In accordance with the double-blind design, Investigators will remain blinded to study treatment and will not have access to the randomization (treatment codes) except under circumstances described in Section 8.7.

Patients will be randomized to one of the treatment arms via a centralized randomization system using an IVRS. A patient will be considered randomized when the treatment number has been provided by the IVRS.

At the screening visit, Visit 1, the site coordinator will contact the IVRS to obtain a patient number for each patient who gives informed consent. Each patient will be allocated a patient number associated with the center and allocated in chronological order in each center.

The treatment assignment will be allocated to the patient according to the central randomization list via the IVRS stratified by region and number of previous anti-TNFs (1 versus > 1). At Visit 2 (Day 1), after confirming the patient is eligible for entry into the treatment period, the site coordinator will contact the IVRS in order to receive the first treatment assignments (kit numbers). Patients will be randomized to receive either one of the 2 treatment arms of sarilumab or its matching placebo. The randomization ratio is 1:1:1 (sarilumab 150 mg: sarilumab 200 mg : matching placebo). At subsequent dispensation visits during the treatment period, the site coordinator will call IVRS to obtain the subsequent treatment kits assignment. A confirmation fax/e-mail will be sent to the site after each assignment.

A randomized patient is defined as a patient who is registered and assigned a randomization number from the IVRS, as documented from the IVRS log file. IMP will also be recorded and tracked on the center IMP inventory forms.

### Example 3. Positive Results with Sarilumab in First Phase 3 Rheumatoid Arthritis Registration Trial

Sarilumab, given subcutaneously every other week, met all three co-primary endpoints.

Sarilumab is the first fully-human monoclonal antibody directed against the Interleukin-6 Receptor (IL-6R).

The results of the SARIL-RA-MOBILITY Phase 3 clinical trial in adult patients with active rheumatoid arthritis (RA) and who were inadequate responders to methotrexate (MTX) therapy show that sarilumab treatment in combination with MTX improved disease signs and symptoms as well as physical function, and inhibited progression of joint damage.

The 52 week SARIL-RA-MOBILITY Phase 3 trial enrolled approximately 1,200 patients with active, moderate-to-severe rheumatoid arthritis, and who were inadequate responders to MTX therapy. Patients were randomized to one of three subcutaneous treatment groups, all in combination with MTX and dosed every other week: sarilumab 200 milligrams (mg), sarilumab 150 mg, or placebo.

Both sarilumab groups showed clinically relevant and statistically significant improvements compared to the placebo group in all three co-primary endpoints (p<0.0001).
(1) Improvement in signs and symptoms of RA at 24 weeks, as measured by the American College of Rheumatology score of 20 percent improvement (ACR20).
   - 66 percent, 58 percent, and 33 percent in sarilumab 200 mg, sarilumab 150 mg, and placebo groups respectively, all in combination with MTX.
(2) Improvement in physical function, as measured by change from baseline in the Health Assessment Question-Disability (HAQ-DI) at week 16.
(3) Inhibition of progression of structural damage at Week 52, as measured by the change in the modified Van der Heijde total Sharp score (mTSS).
   - 0.25, 0.90, and 2.78 in sarilumab 200 mg, sarilumab150 mg, and placebo groups respectively, all in combination with MTX.
   - The group receiving the 200 mg dose of sarilumab + MTX had a reduction of approximately 90 percent in the radiographic progression assessed by the mTSS compared to the radiographic progression with placebo + MTX at week 52.

In the SARIL-RA-MOBILITY trial, there was a higher incidence of treatment emergent adverse events leading to withdrawal in sarilumab treatment groups compared to placebo (13.9 percent in 200 mg, 12.5 percent in 150 mg and 4.7 percent in placebo). In conclusion, IL-6 blockade is an important therapeutic approach for rheumatoid arthritis, because these Phase 3 results, demonstrated efficacy at both doses of sarilumab, each administered every other week.

### Methods:

In Phase 3 of the MOBILITY study, MTX-IR adults with moderate-to-severe RA, were randomized (1:1:1) to placebo and one of two subcutaneous doses of sarilumab (150 mg q2w or 200 mg q2w) plus background MTX. Inclusion and exclusion criteria are shown in Table 2. Patient disposition within the study is provided in Table 3. Baseline characteristics of the subjects are provided in Tables 4 and 5.

**Table 2 Inclusion and Exclusion Criteria**

| **Inclusion criteria** | **Exclusion criteria** |
|---|---|
| •ACR Class I to III RA with duration ≥3 months | • Age <18 or >75 years |
| | • Autoimmune disease other than RA or significant systemic involvement |
| •Moderate-to-severe, active RA based on joint counts and high-sensitivity CRP (hsCRP) | |
| | • Current or recent treatment with DMARDs other than MTX |
| •At risk of structural progression: | • Prior therapy with any other biologic agents within 3 months |
| - Presence of bone erosion or | |
| - Anti-cyclic citrullinated peptide positive (anti-CCP⁺) or | • Use of parenteral or intra-articular glucocorticoids within 4 weeks |
| - Rheumatoid factor positive (RF⁺) | |
| • Methotrexate | |
| - Stable dose for ≥ 6 weeks prior to screening | |
| - ≥ 12 weeks' treatment prior to random ization | |

**Table 3. Patient Disposition**

| **Inclusion criteria** | **Exclusion criteria** |
|---|---|
| • ACR Class I to III RA with duration ≥3 months | • Age <18 or >75 years |
| • Moderate-to-severe, active RA based on joint counts and high-sensitivity CRP (hsCRP) | • Autoimmune disease other than RA or significant systemic involvement |
| • At risk of structural progression: | • Current or recent treatment with DMARDs other than MTX |
| - Presence of bone erosion or | • Prior therapy with any other biologic agents within 3 months |
| - Anti-cyclic citrullinated peptide positive (anti-CCP⁺) or | • Use of parenteral or intra-articular glucocorticoids within 4 weeks |
| - Rheumatoid factor positive (RF⁺) | |
| • Methotrexate | |
| - Stable dose for ≥ 6 weeks prior to screening | |
| - ≥ 12 weeks' treatment prior to random ization | |

**Table 4. Baseline Demographic Characteristics of Subjects**

| **Demographic characteristics** | |
|---|---|
| Age, years, mean | 51 |
| Female % | 82 |
| White % | 86 |
| Means weight, kg | 74 |
| Region, % | |
| N. America, W. Europe, Australia, New Zealand | 17 |
| Latin America | 39 |
| Rest of the world | 43 |

**Table 5. Baseline Rheumatoid Arthritis Characteristics of Subjects**

| **Rheumatoid arthritis characteristics** | |
|---|---|
| Duration of RA, years, mean | 9 |
| Prior biologic use, % | 27 |
| RF+, % | 85 |
| CCP+, % | 87 |
| Tender joint count (0-68), mean | 27 |
| Swollen joint count (0-66), mean | 17 |
| CRP, mg/L, median | 14 |
| DAS-28-CRP, median | 6.0 |

The objective of the study was to evaluate clinical efficacy, radiographic and functional improvement, and safety profile of sarilumab dosing regimens. The radiographic variables of interest included: 1) change from baseline in van der Heijde modified total Sharp score (mTSS) at Week 52 (co-primary endpoint); 2) sensitivity analyses to evaluate impact on mTSS of patients without post-baseline X-ray data; 3) radiographic progression of erosion and joint space narrowing (JSN) scores (subsets of mTSS); 4) proportion of patients with no radiographic progression from baseline to Week 52 in mTSS, erosion, or JSN scores. All radiographic assessments were performed using standardized technique with electronic images analyzed independently by at least 2 trained readers and the average of the 2 scores was used for analysis.

### Results:

At weeks 24 and 52, smaller, statistically significant increases from baseline in mTSS were observed in patients treated with sarilumab compared with placebo, indicating inhibition of structural damage, as shown in Table 6.

**Table 6. Efficacy of sarilumab on clinical, functional, and radiographic outcomes**

| | | Mean Change (SD) from Baseline | | |
|---|---|---|---|---|
| | Week | Placebo + MTX (N=398) | Sarilumab 150 mg q2w + MTX (N=400) | Sarilumab 200 mg q2w + MTX (N=399) |
| mTSS* | 24 | n=338 | n=343 | n=346 |
| | | 1.22 (3.97) | 0.54 (2.99) * | 0.13 (2.60) *** |
| mTSS | 52 | n=352 | n=352 | n=359 |
| | | 2.78 (7.73) | 0.90 (4.66) *** | 0.25 (4.61) *** |
| Erosion Score^{†} | 24 | n=338 | n=343 | n=346 |
| | | 0.68 (2.41) | 0.26 (1.46)* | 0.02 (1.23) *** |
| Erosion Score^{†} | 52 | n=352 | n=352 | n=359 |
| | | 1.46 (4.83) | 0.42 (2.50) *** | 0.05 (2.17) *** |
| JSN Score^{†} | 24 | n=338 | n=343 | n=346 |
| | | 0.54 (2.22) | 0.28 (2.07) | 0.12 (1.82)** |
| JSN Score^{†} | 52 | 1.32 (3.85) | 0.47 (2.88)" | 0.20 (3.21)*** |
| mTSS = the sum of bone erosion scores from 44 joints and JSN scores from 42 joints, with a maximum score of 448. | | | | |
| Erosion Score = the sum of bone erosion scores from 44 joints, with a maximum score of 280. JSN Score = the sum of JSN scores from 42 joints, with a maximum score of 168. | | | | |
| SD = standard deviation | | | | |
| *p-value vs. placebo <0.01; **p-value vs. placebo <0.001; ***p-value vs. placebo <0.0001 | | | | |
| ^{†}Nominal p-values reported for erosion and JSN scores | | | | |

Planned sensitivity analyses for mTSS were consistent with primary results. At Weeks 24 and 52, smaller increases from baseline in the erosion and JSN scores were also observed with sarilumab compared with placebo. The proportion of patients with no progression in mTSS, erosion score, and JSN score was numerically higher in sarilumab groups compared with placebo. Observed adverse events (AEs) were similar to those reported with other IL-6 inhibitors.

Subjects treated with sarilumab and methotrexate showed inter alia improvement in signs and symptoms of RA at 24 and 52 weeks, as measured by the American College of Rheumatology score of 20 percent improvement (ACR20), see table 6b below (asterisk (*) = P<0.0001 vs. Placebo + MTX). These results were 66 percent, 58 percent, and 33 percent in sarilumab 200 mg, sarilumab 150 mg, and placebo groups respectively at 24 weeks and 59 percent, 54 percent, and 32 percent at 52 weeks, all in combination with MTX.

| **Table 6b. ACR efficacy results** | | **Sarilumab** | |
|---|---|---|---|
| | **Placebo + MTX (n=398)** | **150 mg q2w + MTX (n=400)** | **200 mg q2w + MTX (n=399)** |
| ACR20 response - Wk 24, (%) | 33.4% | 58.0%* | 66.4%* |
| ACR50 response - Wk 24, (%) | 16.6% | 37.0%* | 45.6%* |
| ACR70 response - Wk 24, (%) | 7.3% | 19.8%* | 24.8%* |
| ACR20 response - Wk 52, (%) | 31.7% | 53.5%* | 58.6%* |
| ACR50 response - Wk 52, (%) | 18.1% | 40.0%* | 42.9%* |
| ACR70 response - Wk 52, (%) | 9.0% | 24.8%* | 26.8%* |

Subjects treated with sarilumab and methotrexate achieved an ACR70 response for 24 consecutive weeks more often than subjects administered placebo, as shown in Table 7.

**Table 7 ACR70 Response of Subjects**

| | **Placebo + MTX (n=398)** | **Sarliumab + MTX** | |
|---|---|---|---|
| | | **150 mg q2w (n=400)** | **200 mg q2w (n=399)** |
| **Patients who achieved an ACR70 response for at least 24 consecutive weeks, n (%)** | **12 (3.0)** | **51 (12.8)** | **59 (14.8)** |
| | | **P<0.0001** | **P<0.0001** |

Subjects treated with sarilumab and methotrexate also showed improvement in physical function, as measured by change from baseline in the Health Assessment Question-Disability (HAQ-DI) at week 16, as shown in table 8 below.

**Table 8. Efficacy Results**

| **Table. Efficacy results** | | **Sarilumab** | |
|---|---|---|---|
| | **Placebo + MTX (n=398)** | **150 mg q2w + MTX (n=400)** | **200 mg q2w + MTX (n=399)** |
| ACR20 response - Wk 24, n (%) | 133 (33.4%) | 232 (58.0%)* | 265 (66.4%)* |
| ACR50 response - Wk 24, n (%) | 66 (16.6%) | 148 (37.0%)* | 182 (45.6%)* |
| ACR70 response - Wk 24, n (%) | 29 (7.3%) | 79 (19.8%)* | 99 (24.8%)* |
| mTSS, mean change from BL - Wk 52 | 2.8 | 0.9* | 0.3* |
| Major clinical response (ACR70 response maintained for 24 weeks), n (%) | 12 (3.0%) | 51 (12.8%)* | 59 (14.8%)* |

| ACR disease activity measures, adj. mean change from BL - Wk 24 | | | |
|---|---|---|---|
| Swollen joint count | -6.6 | -10.6* | -11.2* |
| Tender joint count | -10.1 | -16.9* | -17.4* |
| HAQ-DI, adj. mean change from BL | | | |
| - Wk 16 | -0.3 | -0.5* | -0.6* |
| - Wk 24 | -0.4 | -0.6* | -0.6* |
| - Wk 52 | -0.5 | -0.7* | -0.8* |

| DAS28-CRP | | | |
|---|---|---|---|
| LS mean change from BL - Wk 24 | -1.17 | -2.45* | -2.82* |
| LS mean change from BL - Wk 52 | -1.36 | -2.78* | -2.95* |
| Clinical remission^{#} - Wk 24, n (%) | 40 (10.1%) | 111 (27.8%)* | 136 (34.1%)* |
| Clinical remission^{#} - Wk 52, n (%) | 34 (8.5%) | 124 (31.0%)* | 136 (34.1%)* |

| CDAI | | | |
|---|---|---|---|
| LS mean change from BL - Wk 24 | -14.47 | -23.89* | -25.79* |
| LS mean change from BL - Wk 52 | -17.50 | -26.96* | -27.26* |
| Remission (≤2.8) - Wk 24, n (%) | 20 (5.0%) | 41 (10.3%)^{†} | 55 (13.8%)* |
| Remission (≤2.8) - Wk 52, n (%) | 19 (4.8%) | 59 (14.8%)* | 72 (18.0%)* |
| No radiographic progression at Wk 52, n (%) | 154 (38.7%) | 191 (47.8%)^{‡} | 222 (55.6%)* |

| | | | |
|---|---|---|---|
| *p<0.0001 vs placebo + MTX; ^{†}p=0.0053; ^{‡}p<0.01 vs placebo + MTX; ^{#}score of <2.6; MTX, methotrexate; BL, baseline; LS, least squared; ACR 20/50/70, American College of Rheumatology 20%/50%/70% improvement criteria; mTSS, van der Heijde modified total Sharp score; HAQ-DI, Health Assessment Questionnaire-Disability Index; HAQ-DI responders, >0.3 improvement in HAQ-DI from baseline; DAS28-CRP, Disease Activity Score in 28 joints using C-Reactive Protein; DAS28-CRP remission, DAS28-CRP <2.6; CDAI, Clinical Disease Activity Index; CDAI remission, CDAI ≤2.8. | | | |

Subjects treated with sarilumab and methotrexate also showed inhibition of progression of structural damage at Week 52, as measured by the change in the modified Van der Heijde total Sharp score (mTSS). 0.25, 0.90, and 2.78 in sarilumab 200 mg, sarilumab150 mg, and placebo groups respectively, all in combination with MTX. The group receiving the 200 mg dose of sarilumab + MTX had a reduction of approximately 90 percent in the radiographic progression assessed by the mTSS compared to the radiographic progression with placebo + MTX at week 52. Subjects treated with sarilumab and methotrexate also showed a decrease in mean DAS28-CRP (Table 8a) and mean CDAI (table 8b) as compared to placebo.

**Table 8a:**

| **DAS28-CRP Remission^{a}, n (%)** | **Placebo + MTX** | **Sarilumab + MTX** | |
|---|---|---|---|
| | | **150mg q2w** | **200mg q2w** |
| **Week 24** | 40 (10.1%) | 111 (27.8%) | 136 (34.1%) |
| | | P<0.0001 | P<0.0001 |
| **Week 52** | 34 (8.5%) | 124 (31.0%) | 136(34.1%) |
| | | P<0.0001 | P<0.0001 |

| | | | |
|---|---|---|---|
| a = DAS28-CRP <2.6; P value vs. placebo + MTX | | | |

**Table 8b:**

| | **Placebo + MTX** | **Sarilumab + MTX** | |
|---|---|---|---|
| | | **150mg q2w** | **200mg q2w** |
| **Mean baseline CDAI⁸** | 40.4 | 40.4 | 40.3 |

| **LS mean difference from baseline** | | | |
|---|---|---|---|
| **Week 24** | -14.5 | -23.9 | -25.8 |
| | | P<0.0001 | P<0.0001 |
| **Week 52** | -17.5 | -27 | -27.3 |
| | | P<0.0001 | P<0.0001 |

| | | | |
|---|---|---|---|
| a = mean for population analyzed at week 24 | | | |

In the SARIL-RA-MOBILITY trial, there was a higher incidence of treatment emergent adverse events leading to withdrawal in sarilumab treatment groups compared to placebo (13.9 percent in 200 mg, 12.5 percent in 150 mg and 4.7 percent in placebo). In conclusion, IL-6 blockade is an important therapeutic approach for rheumatoid arthritis, because these Phase 3 results, demonstrated efficacy at both doses of sarilumab, each administered every other week. This is summarized below in Tables 9-14.

**Table 9. Treatment-Emergent Adverse Events (TEAEs).**

| | **Placebo + MTX** | **Sarilumab + MTX** | |
|---|---|---|---|
| | **(n=427)** | **150 mg q2w** | **200 mg q2w** |
| | **(310 pt yrs)** | **(n=431)** | **(n=424)** |
| | | **(369 pt yrs)** | **(364 pt yrs)** |
| Patient with any TEAE, n (%) | 263 (61.6) | 321 (74.5) | 331 (78.1) |
| Number of TEAEs, (number of TEAEs per 100 patient-years) | 711 (229.6) | 1108 (300.1) | 1236 (339.2) |
| Patient with any SAE, n(%) | 23 (5.4) | 38 (8.8) | 48 (11.3) |
| Number of SAEs, (number of SAEs per 100 patient-years) | 40 (12.9) | 62 (16.8) | 68 (18.7) |
| Deaths, n(%) | 2 (0.5) | 2 (0.5) | 1 (0.2) |
| Discontinuation for TEAE, n(%) | 20 (4.7) | 54 (12.5) | 59 (13.9) |

**Table 10. Most Frequent TEAEs (>5% in Any Treatment Group)**

| | Placebo + MTX | Sarilumab + MTX | |
|---|---|---|---|
| | (n=427) | 150 mg q2w | 200 mg q2w |
| | (310 pt yrs) | (n=431) | (n=424) |
| | | (369 pt yrs) | (364 pt yrs) |
| Patient with any TEAE, n (%) | 263 (61.6) | 321 (74.5) | 331 (78.1) |
| Number of TEAEs, (number of TEAEs per 100 patient-years) | 711 (229.6) | 1108 (300.1) | 1236 (339.2) |
| Patient with any SAE, n(%) | 23 (5.4) | 38 (8.8) | 48(11.3) |
| Number of SAEs, (number of SAEs per 100 patient-years) | 40 (12.9) | 62 (16.8) | 68 (18.7) |
| Deaths, n(%) | 2 (0.5) | 2 (0.5) | 1 (0.2) |
| Discontinuation for TEAE, n(%) | 20 (4.7) | 54 (12.5) | 59 (13.9) |

**Table 11. Serious Adverse Events (SAEs) by System (> 0.5% in Any Treatment Group)**

| Number (%) of patients | Placebo + MTX (n=427) | Sarilumab + MTX | |
|---|---|---|---|
| | (310 pt yrs) | 150 mg q2w (n=431) | 200 mg q2w (n=424) |
| | | (369 pt yrs) | (364 pt yrs) |
| Patient with any SAE(s) | 23 (5.4) | 38 (8.8) | 48 (11.3) |
| Infections and infestations | 10(2.3) | 11(2.6) | 17(4.0) |
| Blood and lymphatic system | 0 | 5(1.2) | 6(1.4) |
| Musculoskeletal | 5(1.2) | 2(0.5) | 5(1.2) |
| Injury | 0 | 3(0.7) | 5(1.2) |
| Renal and urinary disorders | 1(0.2) | 1(0.2) | 3(0.7) |
| Cardiac disorders | 4(0.9) | 2(0.5) | 3(0.7) |
| Respiratory disorders | 1(0.2) | 5(1.2) | 3(0.7) |
| Gastrointestinal disorders | 2(0.5) | 3(0.7) | 3(0.7) |
| Neoplasms | 3(0.7) | 4(0.9) | 2(0.5) |
| Vascular disorders | 1(0.2) | 4(0.9) | 2(0.5) |
| Hepatobiliary disorders | 1(0.2) | 3(0.7) | 2(0.5) |
| Investigations | 0 | 3(0.7) | 2(0.5) |
| Nervous system disorders | 3(0.7) | 1(0.2) | 1(0.2) |

**Table 12. Adverse Events of Special Interest**

| Number (%) of patients | Placebo + MTX (n=427) | Sarilumab + MTX | |
|---|---|---|---|
| | | 150 mg q2w (n=431) | 200 mg q2w (n=424) |
| Serious infections | 10 (2.3) | 11 (2.6) | 17 (4.0) |
| Diverticulitis, gastrointestinal ulceration and perforations* | 2 (0.5) | 8 (1.9) | 4 (0.9) |
| Anaphylaxis | 0 | 0 | 0 |
| Malignancy | 1 (0.2) | 4 (0.9) | 3 (0.7) |
| Lupus-like syndrome (discoid lupus) | 0 | 1 (0.2) | 0 |
| Demyelinating disorders (monoclonal gammopathy) | 1 (0.2) | 0 | 0 |
| MACE (myocardial infarction, stroke, or hospitalization for TIA or unstable angina) | 1 (0.2) | 2 (0.5) | 1 (0.2) |

**Table 13. Incidence: Neutropenia and Thrombocytopenia**

| Number of patients (%) | Placebo + MTX | Sarilumab + MTX | |
|---|---|---|---|
| | (n=427) | 150 mg q2w | 200 mg q2w |
| | (310 pt yrs) | (n=431) | (n=424 |
| | | (369 pt yrs) | (364 pt yrs) |
| Neutropenia | | | |
| Grade 1 (1.5 to <LLN giga/L) | 18 (4.2) | 57 (13.2) | 71 (16.7) |
| Grade 2 (1.0 to <1.5 giga/L) | 6 (1.4) | 56 (13.0) | 70 (16.5) |
| Grade 3 (0.5 to <1.0 giga/L) | 0 | 22 (5.1) | 33 (7.8) |
| Grade 4 (<0.5 giga/L) | 0 | 4 (0.9) | 3 (0.7) |
| Grade 3 to 4 neutropenia | 0 | 26/431 (6.0) | 36/424 (8.5) |
| Leading to treatment discontinuation* | 0 | 9(2.1) | 10 (2.4) |
| Thrombocytopenia | | | |
| ≥50 and <100 giga/L | 0 | 4 (0.9) | 3 (0.7) |
| <50 giga/L | 0 | 0 | 2 (0.5) |
| Leading to treatment discontinuation* | 0 | 1 (0.2) | 2 (0.5) |

**Table 14. Laboratory Data - ALT Elevation**

| **Number of patients (%)** | **Placebo + MTX** | **Sarilumab + MTX** | |
|---|---|---|---|
| | **(n=427)** | **150 mg q2w** | **200 mg q2w** |
| | **(310 pt yrs)** | **(n=431)** | **(n=424)** |
| | | **(369 pt yrs)** | **(364 pt yrs)** |
| ALT >1 to ≤3 ULN | 140 (32.8) | 192 (44.5) | 228 (53.8) |
| ALT >3 to ≤5 ULN | 8 (1.9) | 28 (6.5) | 24 (5.7) |
| ALT >5 to ≤10 ULN | 1 (0.2) | 9 (2.1) | 9 (2.1) |
| ALT >10 to ≤20 ULN | 0 | 4 (0.9) | 1 (0.2) |
| ALT >20 ULN | 0 | 0 | 0 |
| ALT >3 ULN and TB >2 ULN | 0 | 2 (0.5) | 1 (0.2) |

| | | | |
|---|---|---|---|
| ALT >3 ULN and TB >2 ULN: Possible bile duct stone Biliary pancreatitis Occurred ∼10 months after initiation of therapy which investigator attributed to recent chemical exposure. Hepatic mass identified on CT. Hospitalized for drainage of liver abscess 2 months later | | | |

### Conclusion:

Treatment with both doses of sarilumab in combination with MTX was associated with significantly less radiographic progression of structural damage compared with placebo, as measured by mTSS, erosion and JSN scores. Adverse events observed with sarilumab were consistent with IL-6 blockade.

The compositions and methods of the present disclosure are not to be limited in scope by the specific embodiments described herein. Indeed, various modifications of the invention in addition to those described herein will become apparent to those skilled in the art from the foregoing description.

## Claims

1. An anti-IL6R antibody comprising the heavy chain variable region of SEQ ID NO:2 and the light chain variable region of SEQ ID NO:3 for use in combination with methotrexate for inhibiting the progression of structural damage in a subject suffering from rheumatoid arthritis, wherein an effective amount of the antibody and an effective amount of methotrexate are administered to the subject, and wherein the antibody is administered subcutaneously to the subject between 150 and 200 mg per two weeks.

2. The antibody for use in combination with methotrexate according to claim 1, wherein inhibition of the progression of structural damage is **characterised in that** the subject achieves after at least 24 weeks of treatment a change from baseline (BL) in the modified Van der Heijde total Sharp score (mTSS) of at most 0.6.

3. The antibody for use in combination with methotrexate according to claim 1 or 2, wherein inhibition of the progression of structural damage is **characterised in that** the subject achieves after at least 52 weeks of treatment a change from baseline (BL) in the modified Van der Heijde total Sharp score (mTSS) of at most 1.

4. The antibody for use in combination with methotrexate according to any one of claims 1 to 3, wherein the subject was previously ineffectively treated for rheumatoid arthritis by administering methotrexate.

5. The antibody for use in combination with methotrexate according to any of claims 1-4, wherein methotrexate is administered between 6 to 25 mg per week.

6. The antibody for use in combination with methotrexate according to any of claims 1-5, wherein the antibody is administered to the subject for at least 52 weeks.

7. The antibody for use in combination with methotrexate according to any of claims 1-6, wherein inhibition of the progression of structural damage is **characterised in that** the subject achieves after at least 24 weeks of treatment a change from baseline (BL) in the modified Van der Heijde total Sharp score (mTSS) of at most 0.2.

8. The antibody for use in combination with methotrexate according to any of claims 1-7, wherein inhibition of the progression of structural damage is **characterised in that** the subject achieves after at least 24 weeks of treatment a change from baseline (BL) in the modified Van der Heijde total Sharp score (mTSS) of about 0.13.

9. The antibody for use in combination with methotrexate according to any of claims 1-8, wherein inhibition of the progression of structural damage is **characterised in that** the subject achieves after at least 52 weeks of treatment a change from baseline (BL) in the modified Van der Heijde total Sharp score (mTSS) of at most 0.3.

10. The antibody for use in combination with methotrexate according to any of claims 1-9, wherein inhibition of the progression of structural damage is **characterised in that** the subject achieves after at least 52 weeks of treatment a change from baseline (BL) in the modified Van der Heijde total Sharp score (mTSS) of about 0.25.

11. The antibody for use in combination with methotrexate according to any of claims 1-10, wherein the antibody is administered at 150 mg per two weeks.

12. The antibody for use in combination with methotrexate according to any of claims 1-10, wherein the antibody is administered at 200 mg per two weeks.

## Patentansprüche

1. Anti-IL6R-Antikörper, umfassend die variable Region der schweren Kette (VH-Region) der SEQ ID NO: 2 und die variable Region der leichten Kette (VL-Region) der SEQ ID NO: 3 zur Verwendung in Kombination mit Methotrexat, um die Progression der strukturellen Schäden bei einem unter der rheumatoiden Arthritis leidenden Probanden zu hemmen, wobei eine wirksame Menge des Antikörpers und eine wirksame Menge Methotrexat an den Probanden verabreicht werden, und wobei der Antikörper in einer Menge zwischen 150 und 200 mg je zwei Wochen verabreicht wird.

2. Antikörper zur Verwendung in Kombination mit Methotrexat nach Anspruch 1, wobei die Hemmung der Progression der strukturellen Schäden **dadurch gekennzeichnet ist, dass** der Proband nach mindestens 24-wöchiger Behandlung bei der mTSS (modified Van der Heijde total Sharp score) eine Veränderung gegenüber der Basislinie (BL) von höchstens 0,6 erreicht.

3. Antikörper zur Verwendung in Kombination mit Methotrexat nach Anspruch 1 oder 2, wobei die Hemmung der Progression der strukturellen Schäden **dadurch gekennzeichnet ist, dass** der Proband nach mindestens 52-wöchiger Behandlung bei der mTSS (modified Van der Heijde total Sharp score) eine Veränderung gegenüber der Basislinie (BL) von höchstens 1 erreicht.

4. Antikörper zur Verwendung in Kombination mit Methotrexat nach einem der Ansprüche 1 - 3, wobei der Proband vorher eine unwirksame Behandlung für rheumatoide Arthritis erhalten hatte, die in einer Methotrexatgabe bestand.

5. Antikörper zur Verwendung in Kombination mit Methotrexat nach einem der Ansprüche 1 - 4, wobei Methotrexat in einer Menge zwischen 6 und 25 mg wöchentlich verabreicht wird.

6. Antikörper zur Verwendung in Kombination mit Methotrexat nach einem der Ansprüche 1 - 5, wobei der Antikörper mindestens 52 Wochen lang an den Probanden verabreicht wird.

7. Antikörper zur Verwendung in Kombination mit Methotrexat nach einem der Ansprüche 1 - 6, wobei die Hemmung der Progression der strukturellen Schäden **dadurch gekennzeichnet ist, dass** der Proband nach mindestens 24-wöchiger Behandlung bei der mTSS (modified Van der Heijde total Sharp score) eine Veränderung gegenüber der Basislinie (BL) von höchstens 0,2 erreicht.

8. Antikörper zur Verwendung in Kombination mit Methotrexat nach einem der Ansprüche 1 - 7, wobei die Hemmung der Progression der strukturellen Schäden **dadurch gekennzeichnet ist, dass** der Proband nach mindestens 24-wöchiger Behandlung bei der mTSS (modified Van der Heijde total Sharp score) eine Veränderung gegenüber der Basislinie (BL) von etwa 0,13 erreicht.

9. Antikörper zur Verwendung in Kombination mit Methotrexat nach einem der Ansprüche 1 - 8, wobei die Hemmung der Progression der strukturellen Schäden **dadurch gekennzeichnet ist, dass** der Proband nach mindestens 52-wöchiger Behandlung bei der mTSS (modified Van der Heijde total Sharp score) eine Veränderung gegenüber der Basislinie (BL) von höchstens 0,3 erreicht.

10. Antikörper zur Verwendung in Kombination mit Methotrexat nach einem der Ansprüche 1 - 9, wobei die Hemmung der Progression der strukturellen Schäden **dadurch gekennzeichnet ist, dass** der Proband nach mindestens 52-wöchiger Behandlung bei der mTSS (modified Van der Heijde total Sharp score) eine Veränderung gegenüber der Basislinie (BL) von etwa 0,25 erreicht.

11. Antikörper zur Verwendung in Kombination mit Methotrexat nach einem der Ansprüche 1 - 10, wobei der Antikörper in einer Menge von 150 mg je zwei Wochen verabreicht wird.

12. Antikörper zur Verwendung in Kombination mit Methotrexat nach einem der Ansprüche 1 - 10, wobei der Antikörper in einer Menge von 200 mg je zwei Wochen verabreicht wird.

## Revendications

1. Anticorps anti-IL6R comprenant la région variable de chaîne lourde de SEQ ID NO: 2 et la région variable de chaîne légère de SEQ ID NO: 3 destiné à être utilisé en combinaison avec du méthotrexate pour inhiber la progression d'une lésion structurale chez un sujet souffrant de polyarthrite rhumatoïde, où une quantité efficace de l'anticorps et une quantité efficace de méthotrexate sont administrées au sujet, et où l'anticorps est administré par voie sous-cutanée au sujet entre 150 et 200 mg toutes les deux semaines.

2. Anticorps destiné à être utilisé en combinaison avec du méthotrexate selon la revendication 1, où l'inhibition de la progression d'une lésion structurale est **caractérisée en ce que** le sujet présente, après au moins 24 semaines de traitement, par rapport à l'état basal (BL), un changement du score de Sharp total modifié par Van der Heijde (mTSS) de 0,6 au maximum.

3. Anticorps destiné à être utilisé en combinaison avec du méthotrexate selon la revendication 1 ou 2, où l'inhibition de la progression d'une lésion structurale est **caractérisée en ce que** le sujet présente, après au moins 52 semaines de traitement, par rapport à l'état basal (BL), un changement du score de Sharp total modifié par Van der Heijde (mTSS) de 1 au maximum.

4. Anticorps destiné à être utilisé en combinaison avec du méthotrexate selon l'une quelconque des revendications 1 à 3, où le sujet a été précédemment traité sans succès pour la polyarthrite rhumatoïde par une administration de méthotrexate.

5. Anticorps destiné à être utilisé en combinaison avec du méthotrexate selon l'une quelconque des revendications 1 à 4, où le méthotrexate est administré entre 6 et 25 mg par semaine.

6. Anticorps destiné à être utilisé en combinaison avec du méthotrexate selon l'une quelconque des revendications 1 à 5, où l'anticorps est administré au sujet pendant au moins 52 semaines.

7. Anticorps destiné à être utilisé en combinaison avec du méthotrexate selon l'une quelconque des revendications 1 à 6, où l'inhibition de la progression d'une lésion structurale est **caractérisée en ce que** le sujet présente, après au moins 24 semaines de traitement, par rapport à l'état basal (BL), un changement du score de Sharp total modifié par Van der Heijde (mTSS) de 0,2 au maximum.

8. Anticorps destiné à être utilisé en combinaison avec du méthotrexate selon l'une quelconque des revendications 1 à 7, où l'inhibition de la progression d'une lésion structurale est **caractérisée en ce que** le sujet présente, après au moins 24 semaines de traitement, par rapport à l'état basal (BL), un changement du score de Sharp total modifié par Van der Heijde (mTSS) d'environ 0,13.

9. Anticorps destiné à être utilisé en combinaison avec du méthotrexate selon l'une quelconque des revendications 1 à 8, où l'inhibition de la progression d'une lésion structurale est **caractérisée en ce que** le sujet présente, après au moins 52 semaines de traitement, par rapport à l'état basal (BL), un changement du score de Sharp total modifié par Van der Heijde (mTSS) de 0,3 au maximum.

10. Anticorps destiné à être utilisé en combinaison avec du méthotrexate selon l'une quelconque des revendications 1 à 9, où l'inhibition de la progression d'une lésion structurale est **caractérisée en ce que** le sujet présente, après au moins 52 semaines de traitement, par rapport à l'état basal (BL), un changement du score de Sharp total modifié par Van der Heijde (mTSS) d'environ 0,25.

11. Anticorps destiné à être utilisé en combinaison avec du méthotrexate selon l'une quelconque des revendications 1 à 10, où l'anticorps est administré à 150 mg toutes les deux semaines.

12. Anticorps destiné à être utilisé en combinaison avec du méthotrexate selon l'une quelconque des revendications 1 à 10, où l'anticorps est administré à 200 mg toutes les deux semaines.
